# EUROPEAN PATENT SPECIFICATION

(11) **EP 3 512 775 B1**
(45) Date of publication and mention of the grant of the patent: **31.12.2025**
(21) Application number: 17849957.0
(22) Date of filing: 12.09.2017
(51) Int. Cl.: B65B 55/12, A61J 1/00, A61L 2/24, B65B 3/04, A61J 3/07

(54) **APPARATUS AND METHOD FOR MONITORING AND CONTROLLING THE ASEPTIC FILLING AND SEALING OF PHARMACEUTICAL CONTAINERS WITH A PHARMACEUTICAL FLUID USING ROTARY STAGE**
VORRICHTUNG UND VERFAHREN ZUR ÜBERWACHUNG UND STEUERUNG DER ASEPTISCHEN BEFÜLLUNG UND ABDICHTUNG VON PHARMAZEUTISCHEN BEHÄLTERN MIT EINER PHARMAZEUTISCHEN FLÜSSIGKEIT MITHILFE EINES DREHTISCHS
APPAREIL ET PROCÉDÉ DE SURVEILLANCE ET DE COMMANDE DU REMPLISSAGE ASEPTIQUE ET DE L'ÉTANCHÉITÉ DE RÉCIPIENTS PHARMACEUTIQUES RENFERMANT UN FLUIDE PHARMACEUTIQUE À L'AIDE D'UNE PLATINE ROTATIVE

(30) Priority: 13.09.2016 US 201615264554; 21.03.2017 US 201715465516; 12.07.2017 US 201715647633
(43) Date of publication of application: 24.07.2019
(73) Proprietor: VANRX Pharmasystems Inc., Burnaby, BC V5J 5J2 (CA)
(72) Inventor: NAING, Juvenal, Belcarra, British Columbia V3H 5B6 (CA); GOFMAN, Yakov, Richmond, British Columbia V7C 4C7 (CA); CICHY, Marcin, Surrey, British Columbia V3S 3B8 (CA); SENGER, John, New Westminster, British Columbia V3L 4G2 (CA); GUERRERO, Carlos Alberto Diaz, Burnaby, British Columbia V3N 5C4 (CA)
(74) Representative: Fennell, Gareth Charles
(86) International application number: PCT/CA2017/051071
(87) International publication number: WO 2018/049516

(56) References cited:
- EP-A1- 2 192 042
- EP-A2- 2 812 163
- CN-A- 105 271 086
- DE-A1- 102006 039 120
- JP-A- 2012 017 137
- JP-A- H 061 394
- US-A- 3 292 342
- US-A1- 2011 146 841
- US-A1- 2014 196 411
- US-A1- 2016 200 461

## Description

### BACKGROUND OF THE INVENTION

### Field of the Invention

This present invention relates to the medical field as exemplified by IPC class A61 and more particularly to apparatus and associated methods for sterilization of and sterile handling of pharmaceutical materials and containers for pharmaceuticals, including bringing pharmaceuticals into form for administration to medical or veterinary patients. In one aspect, it relates to the programmed and automatic operation of such apparatus.

### Background

The subject of filling pharmaceuticals into pharmaceutical containers is a major aspect of the Pharmaceuticals Industry. The subject is heavily controlled by various governmental and official bodies in various countries. Technologically, the subject is a challenge in that the pharmaceutical products need to be filled into the containers under very strict aseptic conditions. Very specific procedures are specified for this task to a degree that makes the handling of pharmaceuticals profoundly different from the handling of any other industrial product, including specifically semiconductors, which also demand extreme and consistent environmental conditions. Indeed, the parallels between the handling of semiconductors in semiconductor "clean laboratories" and the handling of pharmaceuticals in aseptic isolators are superficial. They share the use of such "clean laboratories", but there is no inherent aseptic requirement associated with semiconductor manufacture.

The filling of pharmaceutical containers with fluid pharmaceuticals specifically requires the aseptic handling of both the containers and the fluid pharmaceutical itself. This leads to complex mechanisms and procedures, many of which may be automated to one degree or another. Often, the production equipment for fluid pharmaceutical handling is bulky and expensive. This creates a problem for smaller operations, particularly in the small-scale production and development environments. As the field has developed, the need for smaller, more compact equipment, particularly in the filling and compounding of fluid pharmaceuticals, has become evident.

The prior art is typically characterized by the use of vibratory bowls and escapements. Many prior art systems also employ gloves for use by the operator to access the interior of the chamber.

EP2192042A1 describes a method involving arranging a plate equipped with vials in a sealed enclosure, and emptying the interior of the enclosure for sucking caps. The caps positioned in complementary tracks of a drawer mounted at the interior of the enclosure are maintained with displacement capacity. The enclosure and the drawer are displaced with the caps for providing free access to the vials. The vials are filled with a product. The drawer is repositioned with the caps above the vials. A pushing effort is exerted on the drawer for tilting the caps in the filled vials.

US3292342A describes closures for combined or co-operating with auxiliary devices for non-closing purposes provided with freeze-drying means.

EP2812163A2 describes a hermetically sealed multi-axis articulated arm apparatus for use within a sealable isolator chamber is disclosed. In one general aspect, it comprises a rotational shaft that passes through an opening in the chamber, a sealing member disposed within the chamber for sealing the shaft to an inner surface of the chamber, interconnected hermetically sealed arm segments operably attached to the linear motion shaft; an end effector operably attached to a terminal arm segment among the arm segments, and at least one fully enclosed drive system for driving and controlling the shaft and the arm segments. The rotational shaft can further be a linear motion shaft for which the sealing member is a bellows. The materials for the parts of the apparatus exposed to the atmosphere of the chamber can be compatible with an aseptic and cleanable environment and the surfaces of the arm segments can be shaped to avoid pooling of contaminants.

JPH061394A describes a method to simultaneously apply a number of caps, by inserting tubes in many holes for erecting tubes, provided in a lower body and fitting caps in an intermediate body having the same numbers of fitting holes to position above the lower body and cover therewith and pressing it at a stroke with a pressing plate in the captioned method for collecting tubes of blood.

### SUMMARY OF THE INVENTION

In one general aspect, the invention features a method for filling nested pharmaceutical containers with a pharmaceutical fluid substance, such as a liquid, solution, or suspension having therapeutic properties. The method includes providing a filling system comprising a sterilizable chamber capable of maintaining an aseptic condition, with the chamber comprising a filling station and a planar rotary stage having a destination fiducial locating structure including constraining surfaces. The method also includes transferring into the chamber at least one container tub sealed by a container tub cover and containing a container nest bearing a plurality of pharmaceutical containers, aseptically sealing the chamber, and establishing an aseptic condition within the chamber. The container nest bearing the plurality of pharmaceutical containers is transferred into the destination fiducial locating structure such that the container nest is held in place by the constraining surfaces, and the pharmaceutical fluid substance is dispensed into at least a portion of the plurality of pharmaceutical containers by operating both the rotary stage and the filling station.

In some embodiments the operating the filling station may include rotating the filling station. The dispensing the pharmaceutical fluid substance may comprise dispensing the pharmaceutical fluid substance on an iterative and serial basis into the containers. Providing a filling system may comprise providing a filing apparatus comprising at least one cover removal station within the chamber, with the transferring into the destination fiducial locating structure the container nest comprising removing the container tub cover from the container tub by operating both the rotary stage and the at least one cover removal station. Operating the at least one cover removal station may comprise rotating the at least one cover removal station. Providing the filling system may comprise providing within the chamber at least one cover removal station having an engagement tool, transferring into the chamber at least one container tub may comprise attaching to the container tub cover a cover removal fixture, and operating the at least one cover removal station may comprise engaging the engagement tool with the cover removal fixture.

The method may further comprise transferring into the chamber a container closure tub sealed by a container closure tub cover and containing at least one container closure nest bearing a plurality of pharmaceutical container closures. The method may further comprise positioning one of the at least one closure nests to align closures in the at least one closure nest with corresponding containers in the container nest, transferring the nests of aligned closures and containers to the ramming station by rotating the rotary stage, and forcing the closures into the corresponding containers. Positioning one of the at least one closure nests may comprise obtaining image information about the one of the at least one closure nest, and positioning the one of the at least one closure nests based on the image information.

Positioning one of the at least one closure nest may comprise applying a vacuum to suction cups, lifting the container closure nest with the suction cups, and operating the rotary stage. Transferring into the destination fiducial locating opening the container nest may comprise applying a vacuum to suction cups, lifting the container nest with the suction cups, and operating the rotary stage. Dispensing the pharmaceutical fluid substance may comprise simultaneously and/or serially operating the rotary stage and the filling station, and removing the container tub cover may comprise simultaneously and/or serially operating the rotary stage and the at least one cover removal station.

In another general aspect, the invention features a system for filling nested pharmaceutical containers with a pharmaceutical fluid substance comprising a sterilizable chamber capable of maintaining an aseptic condition. The chamber includes a filling station, and a planar rotary stage having a rotary stage rotation axis and comprising a destination fiducial locating structure including constraining surfaces disposed and shaped to receive and hold a pharmaceutical container nest bearing a plurality of pharmaceutical containers.

In further embodiments the filling station may comprise a fluid product dispenser head, with the filling station being configured to be rotatable about a filling station rotation axis parallel to the rotary stage rotation axis to position in combination with rotation of the rotary stage the dispenser head over any one of the plurality of pharmaceutical containers held in the container nest in the destination fiducial locating structure. The chamber may further comprise at least one cover removal station and the rotary stage may further comprise a first source fiducial locating structure including constraining surfaces disposed and shaped to receive and hold a pharmaceutical container closure tub sealed by a container closure tub cover and containing at least one pharmaceutical container closure nest bearing a plurality of pharmaceutical container closures, and at least one second source fiducial locating opening disposed and shaped to receive and hold a pharmaceutical container tub sealed by a container tub cover and containing a pharmaceutical container nest bearing a plurality of pharmaceutical containers.

The at least one cover removal station may be disposed and configured to be rotatable about a cover removal station rotation axis parallel to the rotary stage rotation axis to remove in combination with rotation of the rotary stage the container tub cover from the at least one container tub and the container closure tub cover from the container closure tub. At least one cover removal station may comprise an engagement tool disposed and configured to engage with engagement fixtures pre-attached to the container tub cover and to the container closure tub cover.

The system may further comprise at least one camera disposed to obtain image information about at least one of the container nest and the closure nest, and a controller, with the chamber further comprising at least one vacuum pickup system comprising suction cups disposed to engage with the container nests and the container closure nests, the at least one vacuum pickup system being configured in combination with rotation of the rotary stage to lift a pharmaceutical container nest from a pharmaceutical container tub held in one of the at least one second source fiducial locating openings and to deposit the pharmaceutical container nest in the destination fiducial locating opening in combination with rotation of the rotary stage and to lift a pharmaceutical container closure nest from a pharmaceutical container closure tub held in the first source fiducial locating opening and to deposit the container closure nest on top of the pharmaceutical container nest under control of the controller.

The controller may be operative to instruct the at least one camera to provide to the controller the image information and the controller may be operative to control the rotation of the rotary stage to place the closures in the closure nest in correspondence with containers in the container nest. The system may further comprise a ram system configured for forcing the closures into the corresponding containers.

The system may further comprise at least one rotatable cover removal station having a cover removal station rotation axis parallel to the rotary stage rotation axis, at least one vacuum pickup system for placing the container closure nest on the container nest with closures in the closure nest in correspondence with containers in the container nest, and a ram system for forcing the closures into the containers, with the filing station being a rotatable filling station having a filling station rotation axis parallel to the rotary stage rotation axis and comprising a fluid product dispenser head. The system may further comprise at least one camera for obtaining image information of at least one of the container nest and the closure nest, and a controller comprising a memory and a processor. The controller may be operative to instruct the rotary stage to rotate to angular positions that are one of predetermined and based on the image information and to control the at least one cover removal station, the filling station, the at least one vacuum pickup system, and the ram system to operate in conjunction with the rotary stage.

In a further general aspect, the invention features a system for filling nested pharmaceutical containers with a pharmaceutical fluid substance that includes means for establishing and maintaining an aseptic condition in a chamber, means for constraining a container nest bearing a plurality of pharmaceutical containers in the chamber, and means for transferring a container nest to the means for constraining from a container tub in the chamber. It also includes means for rotating the means for constraining in the chamber; and means for dispensing the pharmaceutical fluid substance into at least a portion of the plurality of pharmaceutical containers in the container nest while the container nest is constrained by the means for constraining.

In a further aspect, a system is provided for filling nested pharmaceutical containers with a pharmaceutical fluid substance, the system comprising a sterilizable chamber capable of maintaining an aseptic condition, the chamber comprising: a planar rotary stage having a rotary stage rotation axis, a plurality of locating structures positioned with respect to the rotary stage at different positions around the rotary stage rotation axis, for holding nests of pharmaceutical container parts at the different positions around the rotary stage rotation axis, and a container filling station having a dispensing head for filling the containers while they are held in a nest at one of the locating structures. The locating structures may include surfaces associated with a first tub-holding opening in the rotary stage for holding a first tub containing at least one nest of containers, surfaces associated with a second tub-holding opening in the rotary stage for holding a second tub containing at least one nest of closures, and surfaces associated with a destination nest-holding opening in the rotary stage for holding at least one nest.

The chamber may further comprise at least one vacuum pickup system comprising suction cups disposed to engage with the container nest and container closure nest held on the rotary stage, the at least one vacuum pickup system being configured in combination with rotation of the rotary stage to lift a pharmaceutical container nest from a pharmaceutical container tub and to deposit the pharmaceutical container nest in the destination opening in combination with rotation of the rotary stage and to lift a pharmaceutical container closure nest from a pharmaceutical container closure tub and to deposit the container closure nest on top of the pharmaceutical container nest.

At least one of the locating structures may include a reconfigurable locating structure with one or more adjustable positioning surfaces to position a tub with respect to the rotary stage. The reconfigurable locating structure may include at least one pair of a reconfigurable stopping member and a restraining member disposed opposite each other across an opening in the rotary stage to precisely position at a first predetermined position a tub that contains at least one nest. The stopping member may be adjustable to stop the tub at the first predetermined position by a rotary adjustment and the restraining member may be disposed to restrain the tub in the first predetermined position.

At least a first of the reconfigurable locating structures may include a rotary positioning element having an axis of rotation parallel to a plane of the rotary stage and includes a plurality of different positioning surfaces that are selectable by rotating the rotary positioning element. At least one of the reconfigurable locating structures may include a pair of opposing rotary positioning elements each having an axis of rotation parallel to a plane of the rotary stage and each may include a plurality of different positioning surfaces that are selectable by rotating the rotary positioning elements to accommodate different nest widths.

At least one of the reconfigurable locating structures may include at least a first pair of opposing positioning elements that define positioning surfaces that oppose each other along a first positioning axis that is at least generally parallel to a plane of the rotary stage and at least a second pair of opposing positioning elements that define positioning surfaces that oppose each other along a second positioning axis that is at least generally parallel to a plane of the rotary stage and at least generally perpendicular to the first positioning axis. The at least one of the positioning elements in each of the first and second pairs of positioning elements may include a rotary positioning element having an axis of rotation parallel to a plane of the rotary stage and including a plurality of different positioning surfaces.

The system may further include a reconfigurable vacuum pickup system comprising: a first set of suction cups arranged in a first pattern, a second set of suction cups arranged in a second pattern different from the first pattern, and a selection mechanism operative to position either the first set of suction cups or the second set of suction cups to engage with the at least a first of the nests of pharmaceutical container parts while it is held by one of the plurality of locating structures. The selection mechanism of the reconfigurable vacuum pickup system may include a rotary mechanism operative to position the first or second sets of suction cups in an engagement position.

The system may further include at least one cover removal station positioned to remove covers from tubs containing at least one nest of pharmaceutical packaging materials held in one of the locating structures. The at least one cover removal station may be rotatable about a cover removal station rotation axis parallel to the rotary stage rotation axis to remove the tub covers in combination with rotation of the rotary stage. The at least one cover removal station may comprises an engagement tool disposed and configured to engage with a cover removal fixture on the tub cover.

The filling station may be configured to be rotatable about a filling station rotation axis parallel to the rotary stage rotation axis to position in combination with rotation of the rotary stage the dispenser head over any one of the plurality of pharmaceutical containers held by one of the one of the locating structures.

The system may further comprise at least one camera disposed to obtain image information about at least one of the nests of pharmaceutical container parts. The system may further comprise a ram system configured for forcing nested closures into corresponding nested containers.

The system may further comprise at least one rotatable cover removal station having a cover removal station rotation axis parallel to the rotary stage rotation axis; at least one vacuum pickup system for placing a container closure nest on a container nest with closures in the closure nest in correspondence with containers in the container nest; a ram system for forcing the closures into the containers; and wherein the filing station is a rotatable filling station having a filling station rotation axis parallel to the rotary stage rotation axis and comprising a fluid product dispenser head.

The system may further comprise at least one camera for obtaining image information of at least one of the container nest and the closure nest, a controller comprising a memory and a processor, and wherein the controller is operative to instruct the rotary stage to rotate to angular positions that are one of predetermined and based on the image information and to control the at least one cover removal station, the filling station, the at least one vacuum pickup system, and the ram system to operate in conjunction with the rotary stage.

In another aspect, a system is provided for filling nested pharmaceutical containers with a pharmaceutical fluid substance, comprising: means for establishing and maintaining an aseptic condition in a chamber; means for constraining a container nest bearing a plurality of pharmaceutical containers in the chamber; means for transferring to the means for constraining a container nest from a container tub in the chamber; means for rotating the means for constraining in the chamber; and means for dispensing the pharmaceutical fluid substance into at least a portion of the plurality of pharmaceutical containers in the container nest while the container nest is constrained by the means for constraining.

In a further aspect, a method is provided for filling nested pharmaceutical containers with a pharmaceutical fluid substance, the method comprising: providing a filling system comprising a sterilizable chamber capable of maintaining an aseptic condition, the chamber comprising a filling station and a planar rotary stage having a destination locating structure; transferring into the chamber at least one container tub sealed by a container tub cover and containing a container nest bearing a plurality of pharmaceutical containers; aseptically sealing the chamber; establishing an aseptic condition within the chamber; transferring into the destination locating structure the container nest bearing the plurality of pharmaceutical containers such that the container nest is held in place; and dispensing the pharmaceutical fluid substance into at least a portion of the plurality of pharmaceutical containers by operating both the rotary stage and the filling station. The operating the filling station may include rotating the filling station. The dispensing the pharmaceutical fluid substance may comprise dispensing the pharmaceutical fluid substance on an iterative and serial basis into the containers.

The providing a filling system may comprise providing a filing apparatus comprising at least one cover removal station within the chamber and wherein the transferring into the destination locating structure the container tub comprises removing the container tub cover from the container tub by operating both the rotary stage and the at least one cover removal station. The operating the at least one cover removal station may comprise rotating the at least one cover removal station. The providing the filling system may comprise providing within the chamber at least one cover removal station having an engagement tool, the transferring into the chamber at least one container tub may comprise attaching to the container tub cover a cover removal fixture; and wherein the operating the at least one cover removal station comprises engaging the engagement tool with the cover removal fixture.

The method may further comprise transferring into the chamber a container closure tub sealed by a container closure tub cover and containing at least one container closure nest bearing a plurality of pharmaceutical container closures. The method may further comprise positioning one of the at least one closure nests to align closures in the at least one closure nest with corresponding containers in the container nest; transferring the nests of aligned closures and containers to a ramming station by rotating the rotary stage; and forcing the closures into the corresponding containers. The method may further include adjusting a tub locating structure to accommodate a size of the closure nest tub. The positioning one of the at least one closure nest may comprise: obtaining image information about the one of the at least one closure nests; and positioning the one of the at least one closure nests based on the image information. The positioning one of the at least one closure nest may comprise: applying a vacuum to suction cups; lifting the container closure nest with the suction cups; and operating the rotary stage.

The transferring into the destination locating opening the container nest may comprise: applying a vacuum to suction cups; lifting the container nest with the suction cups; and operating the rotary stage. The method may further include selecting one of a plurality of sets of suction cups and wherein the applying a vacuum to suction cups is performed for the selected set of suction cups. The selecting may include rotating one of the plurality of sets of suction cups into position. The method may further include the destination locating structure to accommodate a size of the container nest. The adjusting may be performed in two at least generally orthogonal directions. The method may further include adjusting a tub locating structure to accommodate a size of the container nest tub.

In another general aspect, the invention features a container assembly for holding nested pharmaceutical container parts. It includes a container comprising a bottom, a top lip that provides a horizontal top sealing surface that has a peripheral outline, and sidewalls located between the bottom and the top lip. It also includes a peelable container cover consisting of a sheet of flexible material sealed to the sealing surface of the top lip of the rectangular container to seal the contents of the container, and a cover removal fixture on the container cover.

The sealed peelable container cover may include a portion that extends outside of the peripheral outline of the top sealing surface of the container, and the cover removal fixture may be on the portion of the peelable container cover that extends outside of the peripheral outline of the top sealing surface of the container. The container may be rectangular and includes four sidewalls. The cover removal fixture may include an appendage to allow it to be engaged by an engagement tool. The cover removal fixture may include a ball-shaped appendage to allow it to be engaged by an engagement tool. The peelable container cover may be heat sealed to the sealing surface of the top lip of the rectangular container to seal the contents of the container against decontamination. The peelable container cover may be sealed to the sealing surface of the top lip of the rectangular container to seal the contents of the container against decontamination using a chemical agent. The peelable container cover may sealed to the sealing surface of the top lip of the rectangular container to seal the contents of the container against decontamination using a radiation. The peelable container cover may be sealed to the sealing surface of the top lip of the rectangular container to seal the contents of the container against decontamination using plasma. The peelable cover may be made of a plastic material. The peelable cover may be made of an impermeable laminated foil. The peelable cover may be made of a polymeric membrane. The cover removal fixture may be clipped to a portion of the peelable container cover that extends outside of the peripheral outline of the top sealing surface of the container. The sealed container may hold sterilized pharmaceutical containers or closures.

In a further aspect, a method is provided for removing within a controlled environment enclosure a container cover from a sealed container, the sealed container being sealed by the container cover, the method comprising: providing the container in the controlled environment enclosure with the cover sealed to a sealing surface of a lip of the container to seal the contents of the container against decontamination, the cover having a cover removal fixture, decontaminating the sealed container in the controlled environment enclosure, engaging the cover removal fixture with an engagement tool, and removing the cover from the container using the engagement tool. The engaging may engage the cover removal fixture with a fork-shaped engagement tool. The engaging may engage a ball-shaped appendage on the cover removal fixture.

The providing may include providing sterilized pharmaceutical containers or closures in the sealed container before the decontaminating. The attaching may take place before the container is in the controlled environment enclosure. The decontaminating the sealed container in the controlled environment enclosure may take place before the removing the cover. The removing the cover may include moving the engagement tool relative to the container. The removing the cover may include moving both the container and the engagement tool. The method may further comprise attaching the cover removal fixture to the cover before providing the container in the controlled environment enclosure.

In a further aspect, a method is provided for removing within a sterilizable environment a cover from a tub sealed with the cover, the method comprising: providing a sterilizable chamber capable of maintaining an aseptic condition; holding in the chamber a tub sealed with a peelable cover, the tub and cover having corresponding pluralities of corners; illuminating one or more peeling monitor zones proximate the cover removal station with light from at least one light source; peeling the cover from one of the plurality of corners of the tub; measuring a first intensity of light from the illuminating at one of the peeling monitor zones proximate to and substantially non-overlapping with the tub and after at least a portion of the peeling; assessing a current status of the peeling of the cover from the tub based on the first intensity of light; and providing an indication of the assessed current status. The measuring a first intensity of light may comprise measuring a first intensity of light from the light source diffusely reflected from the one of the peeling monitor zones.

The method may further comprise deciding a next step in the removing process based on the current status of the peeling. The assessing a current status of the peeling of the cover from the one of the plurality of corners of the tub may comprise comparing the first light intensity with a first predetermined light intensity value. The assessing a current status of the peeling of the cover from the one of the plurality of corners of the tub may comprise detecting the cover blocking light from the illuminating in the one of the peeling monitor zones.

The method may further comprise, if the one of the plurality of corners of the tub is the last of the plurality of corners being peeled, detaching the cover completely from the tub; measuring a last intensity of light from the illuminating at the one of the peeling monitor zones; and assessing the detaching of the cover from the tub based on the last intensity of light and the first intensity of light. The assessing the detaching of the cover from the tub may comprise comparing the last intensity of light with a last predetermined light intensity value. The measuring the last intensity of light may comprise measuring light from the light source diffusely reflected from the one of the peeling monitor zones.

The peeling monitor zones may on a movable platform and the peeling the cover from the one of the plurality of corners of the tub may comprise moving the platform. The providing the sterilizable chamber may comprise providing a sterilizable chamber comprising a planar rotary stage having a source locating structure to perform the holding of the tub, and the peeling the cover from the one of the plurality of corners of the tub may comprise rotating the planar rotary stage. The providing the sterilizable chamber may comprise providing a movable platform having a source locating structure to perform the holding of the tub, and the peeling the cover from the one of the plurality of corners of the tub may comprise moving the platform.

The providing the chamber may comprise providing a cover removal station comprising an engagement tool and the peeling may comprise: attaching a cover removal fixture to the tub cover before placing the tub in the source locating structure; engaging the cover removal fixture with the engagement tool; and moving the engagement tool. The providing the sterilizable chamber may comprise providing a sterilizable chamber comprising a planar rotary stage having a source locating structure to perform the holding of the tub, and the peeling the cover from the one of the plurality of corners of the tub may comprise rotating the planar rotary stage.

In a further aspect a system is provided for automatically monitoring and controlling the removing within a sterilizable environment of a cover sealed to a tub, the tub and cover having corresponding pluralities of corners, the system comprising: a sterilizable chamber capable of maintaining an aseptic condition, the chamber comprising: a source locating structure disposed to hold the tub in a fixed position, a cover removal station disposed to engage with the cover and to peel the cover from the tub, a peeling monitor surface positioned proximate the cover removal station, one or more light sources disposed to illuminate at least a portion of the peeling monitor surface, and one or more light sensors sensitive to light from the light source and disposed to preferentially collect and measure light diffusely reflected from the illuminated portion of the peeling monitor surface. The system may further comprise a controller in communication with the cover removal station, the light source and the light sensor. The light source may be an infrared light source and the sensor may be an infrared sensor.

The controller may comprise a memory and software instructions which when loaded in the memory and executed by the controller cause the controller to operate the cover removal station, the light source and the light sensor. The peeling monitor surface may be part of a surface of a movable platform; and the software instructions when loaded in the memory and executed by the controller may further cause the controller to be able to move the platform. The movable platform may be a planar rotary stage having a rotary stage rotation axis. At least one of the cover removal station and the platform may operable to peel the cover from one of the plurality of corners of the tub and to move a peeled portion of the cover into a peeling monitor zone, the peeling monitor zone being within the illuminated portion of the platform, proximate the tub and substantially non-overlapping with the tub.

The sensor may be configurable for measuring a first intensity of light from the light source diffusely reflected from the peeling monitor zone; and the software instructions when loaded in the memory and executed by the controller may further enable the controller to: operate at least one of the light sources and at least one of the sensors to measure the first intensity of light immediately after the peeled portion of the cover has been moved into the peeling monitor zone; assess a current status of the peeling of the cover from the tub based on the first intensity of light; and determine a next step in the removing based on the current status of the peeling. A first predetermined light intensity value may be stored in the memory and the software instructions when loaded in the memory and executed by the controller may further enable the controller to assess a current status of the peeling of the cover from the tub by comparing the first light intensity with the first predetermined light intensity value.

If the first of the plurality of corners of the tub is the last of the plurality of corners being peeled the software instructions when loaded in the memory and executed by the controller may further enable the controller to: operate at least one of the cover removal station and the platform to detach the cover completely from the tub; operate at least one of the light sources and at least one of the sensors to measure a last intensity of light from the light source diffusely reflected from the peeling monitor zone; and assess the detaching of the cover from the tub based on the last intensity of light and the first intensity of light. A last predetermined light intensity value may be stored in the memory; and the software instructions when loaded in the memory and executed by the controller may further enable the controller to assess the detaching of the cover from the tub by comparing the last light intensity with the last predetermined light intensity value.

Systems and methods according to the invention need not employ either vibratory bowls or escapements. Nor do such systems or method require gloves. Systems and methods according to the invention may therefore address needs for compact, small-scale filling and compounding of fluid pharmaceuticals.

### BRIEF DESCRIPTION OF THE DRAWINGS

The above-mentioned and other features and objects of this invention, and the manner of attaining them, will become more apparent and the invention itself will be better understood by reference to the following description of an embodiment of the invention taken in conjunction with the accompanying drawings, wherein:
**FIG. 1A** is a drawing of an apparatus for filling pharmaceutical containers with a pharmaceutical fluid product. For the sake of clarity some surfaces are shown in cutaway form and others are shown as transparent.
**FIG. 1B** is a plan view of one chamber of the apparatus of **FIG. 1A****.**
**FIG. 1C** shows a rotary stage of the apparatus of **FIG.1A** and **FIG.1B****.**
**FIG.1D** shows a side view of a portion of the apparatus of **FIG.1A** and **FIG.1B****.**
**FIG. 1E** shows a pharmaceutical container tub cover seated in the rotary stage of **FIG.1A** to **FIG.1D** being removed.
**FIG.1F** shows pharmaceutical containers being filled with a pharmaceutical fluid substance in the apparatus of **FIG.1A** to **FIG.1E****.**
**FIG.1G** provides a more detailed view of the cover removal components of the apparatus of **FIG.1A****,** **FIG.1B** and **FIG.1E****.**
**FIG.2A** and **FIG. 2B** jointly form a drawing of a flow chart for a method of aseptically filling pharmaceutical containers with a pharmaceutical fluid substance in a spatially constrained environment.
**FIG.3A** is a drawing of subsystems of another embodiment of an apparatus for filling pharmaceutical containers with a pharmaceutical fluid product.
**FIG.3B** shows a portion of **FIG.3A** in more detail.
**FIG.4A** is a drawing of subsystems of a further embodiment of an apparatus for filling pharmaceutical containers with a pharmaceutical fluid product.
**FIG.4B** shows a portion of **FIG.4A** in more detail.
**FIG.5A** is a drawing of subsystems of yet a further embodiment of an apparatus for filling pharmaceutical containers with a pharmaceutical fluid product.
**FIG.5B** shows a portion of **FIG.5A** in more detail.
**FIG.6** shows a flow chart of a further method for filling nested pharmaceutical containers with a pharmaceutical fluid substance.
**FIG.7** is a drawing of the apparatus of **FIG.1A-G** additionally equipped with a light source and a further sensor.
**FIG.8A-D** is a set of drawings showing the peeling in the system of **FIG.7** of a cover from a tub sealed by the cover along with the use of the light source and further sensor to monitor and control the peeling of the cover.
**FIG.9** shows a flow chart providing more detail of the peeling step of the flow chart in **FIG. 2A** as performed using the system of **FIG.7** and **FIG.8A-D.**

Corresponding reference characters indicate corresponding parts throughout the several views. Although the drawings represent embodiments of the present invention, the drawings are not necessarily to scale and certain features may be exaggerated in order to better illustrate and explain the present invention. The flow charts are also representative in nature, and actual embodiments of the invention may include further features or steps not shown in the drawings. The exemplifications set out herein illustrate embodiments of the invention, in one or more forms, and such exemplifications are not to be construed as limiting the scope of the invention in any manner.

### DETAILED DESCRIPTION

The embodiments disclosed below are illustrative and not intended to be exhaustive or limit the invention to the precise form disclosed in the following detailed description. Rather, the embodiments are chosen and described so that others skilled in the art may utilize their teachings.

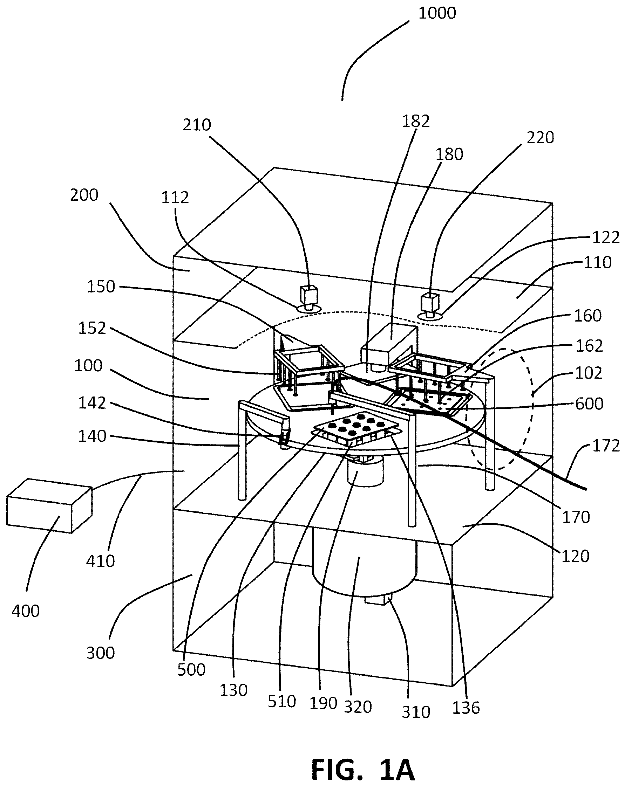

The present invention relates to an apparatus and method for filing pharmaceutical containers with a pharmaceutical fluid substance in a spatially constrained environment. In **FIG.1A****,** filling system **1000** comprises sealable chamber **100** in communication with an ambient environment, sealable chamber **100** being capable of having an aseptic environment established within its interior and capable of maintaining that aseptic environment within its interior. The interior of sealable chamber **100** may be rendered aseptic by any one or more of a number of treatments, including but not limited to treatment with a sterilant, such as steam, hydrogen peroxide vapor, ozone, nitrogen dioxide, and ethylene oxide. The structures and mechanisms to perform such sterilization steps are well known in the art and are not shown in **FIG.1A****.**

Chambers **200** and **300** are separated from chamber **100** by upper wall **110** and lower wall **120** respectively and are not required to be capable of maintaining aseptic environments within their interiors. The communication of chamber **100** with the ambient environment may be via suitable aseptically sealable access door **102,** schematically shown in broken outline in **FIG.1A****.** Suitable sealable doors and ports are well known in the art and will not be dwelt upon further in this specification. The ambient environment may be, for example, a clean room adapted for the handling of pharmaceuticals during production. Since space is at a premium in such spatially constrained clean environments, there is much merit in reducing the so-called "footprint" of equipment to be housed in the clean environment.

The terms "aseptic" and "sterilize" and their derivatives are to be understood as follows for the purposes of the present specification. Establishing an aseptic condition in the interior of a chamber shall be understood to mean establishing that condition throughout the internal atmosphere of the chamber as well as on substantially all exposed interior surfaces of the chamber. This shall include the surfaces of all items, containers, subsystems and the like exposed to the interior atmosphere of the chamber. To the extent that extremely tight crevices or microscopic crevices may exist in the interior of the chamber such that a sterilizing gas or vapor may not perfectly penetrate into such tight regions, for example, the degree of sterilization in practical cases may not be total. This is acknowledged in both the industry and in the standards set for the industry. The action of establishing an aseptic condition within the interior of the chamber and "sterilizing the interior of the chamber" shall have the same meaning in this specification.

Introducing into the interior of a chamber with an aseptic condition an item of which the surfaces are not suitably sterilized destroys the existing aseptic condition within the chamber. Conversely, introducing an aseptic or sterilized item into an interior of a chamber that does not have an aseptic condition within that interior does not render that interior aseptic. In fact, all it does is to destroy the aseptic condition of the surface of the item so introduced. Similarly, introducing filtered air, even with all biological entities filtered out, into an unsterilized chamber does not in any way sterilize the chamber or render it aseptic to a degree acceptable in the pharmaceutical industry. The reason is that the interior surfaces of the chamber are not sterilized by the introduction of such air. All that is achieved is to contaminate the filtered air with active biological species resident on the interior surfaces of the unsterilized chamber.

In the interest of clarity and completeness, it should also be recorded that in the art the term "aseptic" is also sometimes used in association with the introduction of pharmaceutical fluids along aseptic tubes into bodies within controlled chambers. In such cases the term in the art refers to the condition inside the tube or to the fact that the pharmaceutical fluid may be filtered to a suitable degree. This in no way sterilizes or renders aseptic the interior of the chamber in question. The aseptic condition in such cases is confined to the interior of the tube bearing the pharmaceutical stream. Such streams are often filtered to a high degree, but such filtering affects only the interior of the particular tube and does not in any way sterilize the interior of the chamber.

In some prior art systems, containers introduced into a chamber for the purposes of being filled with a pharmaceutical are routed through sterilizing subsystems. This kills biological species on the containers. When such sterilized containers are introduced into the chamber when the chamber itself is not aseptic the containers lose their aseptic condition as biological species contained within the chamber will deposit on the previously aseptic containers.

It should also be pointed out that pharmaceutical or semiconductor clean rooms of any quality level, including "Class 100", "Class 10" or "Class 1", even when employing laminar flow hoods and the like or any quality of HEPA (High Efficiency Particulate Air) filters or ULPA (Ultra Low Particulate Air) filters, cannot constitute an aseptic chamber because they do not have an assurable means to render the surfaces of the room sterile or aseptic. Standards for clean rooms exist from both the United States Federal Government and ISO (International Standards Organization). These specify in great detail to different standards the allowed particulate content of a cubic volume of air in such a clean room facility. None of these standards address the matter of biological species present on surfaces in the room. This serves to make the point that a chamber cannot be rendered aseptic by the management of its atmosphere or airflow only. Nor, conversely, may the chamber be rendered aseptic by the sterilization of only the surfaces of its interior.

The text "Guideline for Disinfection and Sterilization in healthcare Facilities, 2008" by Rutala et al from the Center for Disease Control lists a compendium of mechanisms and methods for sterilization. Our concern in this specification is specifically with those mechanisms for sterilizing the interior of a chamber; that is, sterilizing both the interior surfaces and the atmosphere within the chamber. Given the requirements, vapor base methods are most appropriate to the task. These include, but are not limited to, treatment with heated water vapor, hydrogen peroxide vapor, ozone, nitrogen dioxide, ethylene oxide, glutaraldehyde vapor or other suitable sterilizing gases and vapors. In one suitable method appropriate to the present invention, the sterilization is by means of hydrogen peroxide vapor which is then flushed using ozone before the chamber is employed in the filling of pharmaceutical containers.

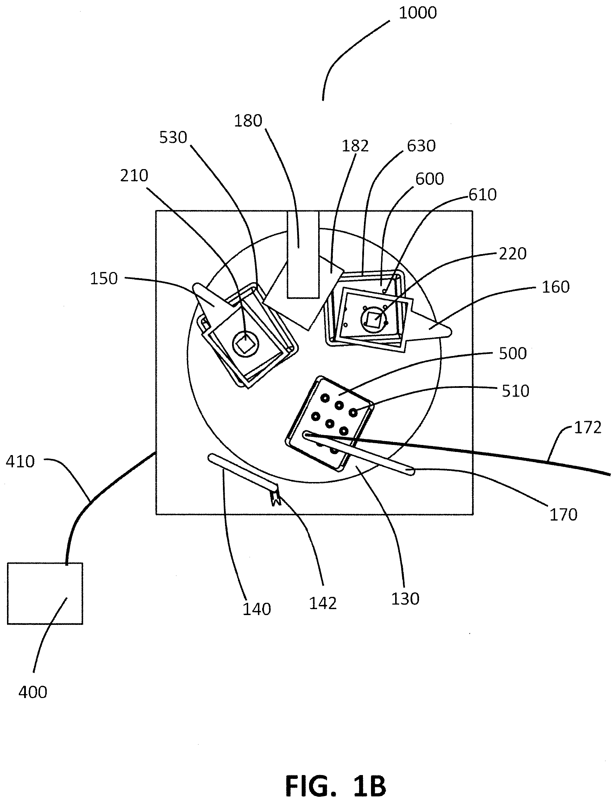

The subsystems of the apparatus **1000** contained with sealable chamber **100** will now be described at the hand of **FIG.1A** to **FIG.1G****.** Due to the compactness and density of components and subsystems of apparatus **1000,** certain components and subsystems are omitted from the drawings of **FIG.1B** to **FIG.1G** in the interest of clarity and the focus is placed on components and subsystems most relevant to the supporting text in this specification. Planar rotary stage **130** is fully rotatable through 360 degrees in a horizontal plane parallel to lower wall **120** about rotary stage rotation axis **131** and may be raised and lowered by means of bellows feed-through **190.** The use of bellows feed-through **190** allows chamber **100** to retain its aseptic condition during the motion of rotary stage **130.** A suitable engine and gearing system **320** may be housed within chamber **300.** Engines, for example stepper motors, as well as gearing systems suitable for rotating rotary stage **130** with suitable angular precision and repeatability are well known in the art and are not further discussed in this specification.

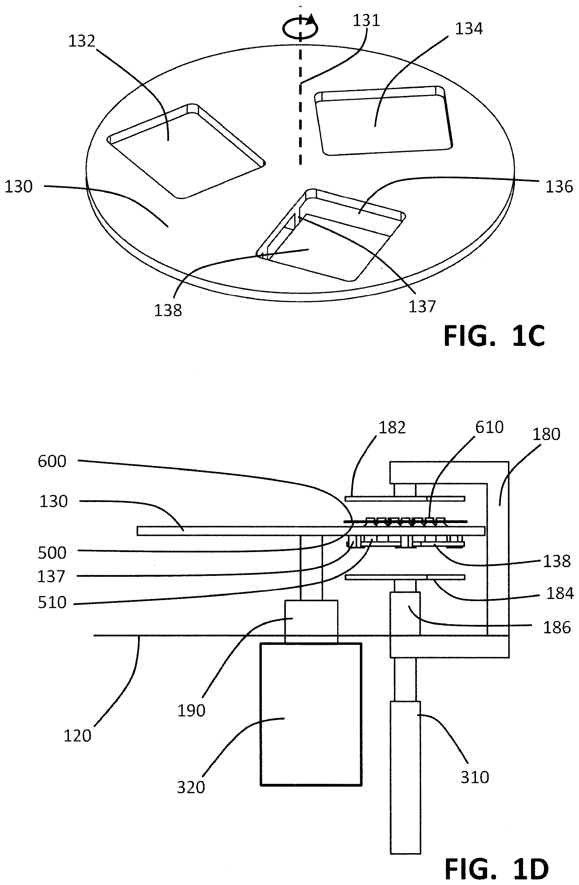

As shown in **FIG. 1C****,** at least three fiducial locating openings **132, 134,** and **136** are provided in rotary stage **130.** Fiducial locating opening **132** is employed for receiving container tubs **530** holding sterilized pharmaceutical containers **510** pre-packed in a predetermined pattern in container nests **500.** Container tubs **530** are typically substantially rectangular and are sealed with peelable covers **520.** Suppliers of pharmaceutical containers provide their product in this format to users of the apparatus of the present specification. Fiducial locating opening **134** is employed for receiving container closure tubs **630** holding sterilized pharmaceutical containers closures **610** pre-packed in a predetermined pattern in container closure nests **600.** Container closure tubs **630** are typically substantially rectangular and are sealed with peelable tub covers not shown in **FIG. 1A** to **FIG. 1G****.** The peelable covers of tubs **630** are functionally identical to peelable covers **520.** Suppliers of pharmaceutical containers provide their product in this format to users of the apparatus of the present specification. In the interest of the compactness of system **1000,** the rectangular axes of locating openings **132, 134,** and **136** may be oriented at an angle with respect to the radial direction of rotary stage **130** in order to ensure a suitably small radius for rotary stage **130.**

Suitable container nests **500** and container closure nests **600;** container tubs **530** and container closure tubs **630;** and peelable tub covers **520** are described in published United States Patent Application US 2016-0200461, the specification of which is hereby incorporated in full. Alternative cover gripping arrangements for the removal of tub covers from tubs are also described in published United States Patent Application US 2016-0251206, the specification of which is hereby incorporated in full.

In the interest of clarity, **FIG. 1A** to **FIG.1G** show, and the associated text to follow below will describe, the use of single tub **530** of pharmaceutical containers **510** along with single tub **630** of container closures **610.** In practice, container closures **610** are provided as multiple nests **600** per container closure tub **630.** To this end rotary stage **130** may contain more than one fiducial locating opening **132** to each receive a respective one of container tub **530** holding sterilized pharmaceutical containers **510** pre-packed in container nest **500.** In yet other implementations, more than one nest **500** of containers **510** may be present in a single pharmaceutical container tub **530.**

Fiducial locating opening **136** is specifically arranged to receive container nests **500** bearing pharmaceutical containers **510.** Whereas tubs **530** and **630** naturally locate in fiducial locating openings **132** and **134** and are suspended by their own rims once in opening **132** and **134,** containers **510** are correctly located in opening **136** and retained in position by some other mechanism. To this end, fiducial locating opening **136** comprises four fiducial retaining guides **137.** Baseplate **138** is located within fiducial locating opening **136** as a loose component of system **1000,** and rests on the horizontal portions at the bottoms of each of four fiducial retaining guides **137** (see **FIG.1C** and **FIG.1D****).** This arrangement allows baseplate **138** to move freely, guided by fiducial retaining guides **137.** We shall return to this arrangement when discussing the closing of containers with container closures.

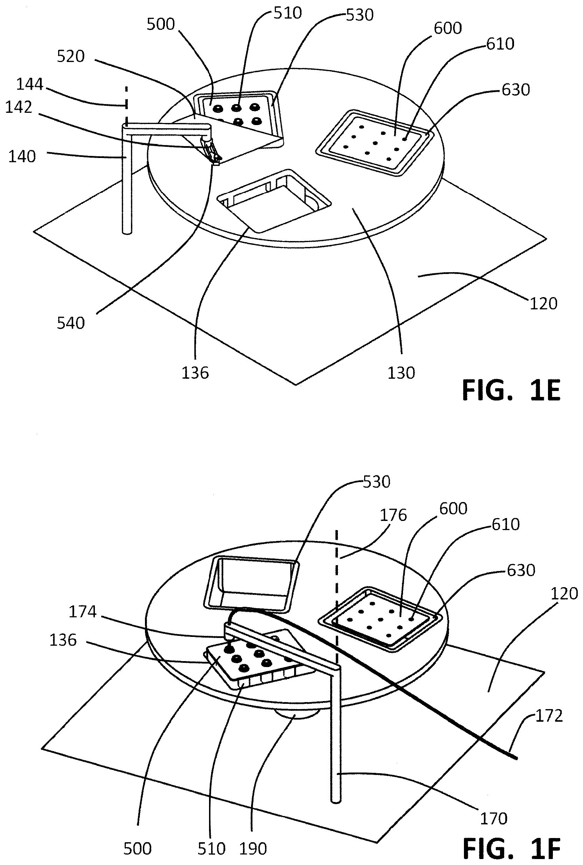

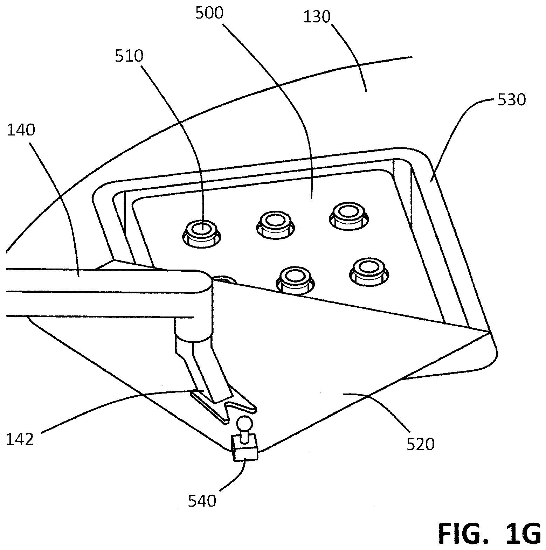

**FIG.1E** shows fiducial locating opening **136** as empty, while cover **520** is being peeled from container tub **530** in fiducial locating opening **132** (not visible) to expose nest **500** bearing pharmaceutical containers **510.** At this point in the operation of system **1000,** a cover similar to cover **520** has already been pealed from tub **630** in fiducial locating opening **134** (not visible) to expose nest **600** bearing container closures **610.** **FIG.1G** shows a close-up detailed view of the peeling of cover **520.** Cover removal station **140** is rotatable about cover removal station rotation axis **144** parallel to rotary stage rotation axis **131** and comprises engagement tool **142,** which, in this particular embodiment, is fork-shaped in order to engage with cover removal fixture **540** attached to cover **520.** Cover removal fixture **540** is pre-attached to cover **520** before tub **530** is transferred into system **1000** via door **102** (See **FIG.1A****).** In the embodiment shown in **FIG.1E** and **FIG.1G****,** cover removal fixture **540** is clipped to cover **520** and has a ball-shaped appendage to allow it to be engaged by engagement tool **142.** Other combinations of cover removal fixtures and engagement tools are contemplated and system **1000** is not limited to the particular combination of cover removal fixture and engagement tool shown in **FIG.1A****,** **FIG.1E** and **FIG.1G****.** Cover removal fixture **540,** for example, may be manufactured as an integral part of cover **520** for use in filling systems such as filling system **1000.** Or it may be clipped to cover **520** during the placement into tub **530** of nests **500** bearing containers **530** and during the placement into tub **630** of nests **600** bearing container closures **610.**

Rotary stage **130** may be lowered to assist in obtaining a less acute angle between cover **520** and tub **530.** Too acute an angle may lead to the tearing of cover **520.** Cover removal station **140** may be rotated while rotary stage **130** rotates so that the combined motions of cover removal station **140** and rotary stage **130** provide a low stress path for the removal of cover **520,** thereby limiting the chances of tearing of cover **520.** In particular, cover removal station **140** may be rotated to ensure that engagement tool **142** is not present above fiducial locating opening **132** when container tub **530** is placed in or removed from fiducial locating opening **132.**

In some embodiments, system **1000** comprises single cover removal station **140** for sequentially removing covers from tubs **520** and **620.** In other embodiments, system **1000** may be equipped with two or more cover removal stations **140** for dedicated removal of covers from tubs **520** and **620** and other additional tubs. In some embodiments covers are simultaneously removed from tubs **520** and **620** and from other tubs, all the removal processes benefiting from a single rotary motion of rotary stage **130.**

In **FIG.1A****,** **FIG.1B****,** and **FIG.1F** filling station **170** for filling pharmaceutical containers **510** with pharmaceutical fluid product comprises pharmaceutical fluid product feed line **172** supplying pharmaceutical fluid product to a pharmaceutical fluid product dispenser head **174** (See **FIG.1F****).** Filling station **170** is rotatable about filling station rotation axis **176** parallel to rotary stage rotation axis **131.** Filling station **170** and rotary stage **130** may simultaneously or sequentially rotate to place dispenser head **174** over an opening of any selected container **510** in nest **500** when nest **500** is seated in fiducial locating opening **136.** This allows every container **510** in nest **500** to be filled with pharmaceutical fluid product by product dispenser head **174.** When not engaged in filling containers **510,** filling station **170** may be rotated to swing dispenser head **174** completely away from fiducial locating opening **136,** thereby allowing nests **600** bearing container closures **610** to be placed on top of nest **500** with closure **610** directly on top of an opening of every container **510** residing in fiducial locating opening **136.**

Another term employed to describe dispenser head **174** is "filling needle". Suitable filling needles and protective sheathing arrangements for such filling needles are described in published United States Patent Applications US 2016-0346777 and US 2017-0121046.

**FIG.1A** and **FIG.1B** show two vacuum pickup systems **150** and **160,** each respectively comprising a plurality of suction cups **152** and **162** (See **FIG.1B****).** Vacuum pickup system **150** is arranged to pick up nests **500** of containers **510** by means of suction cups **152,** and vacuum pickup system **160** is arranged to pick up nests **600** of containers **610** by means of suction cups **162.** Vacuum pickup system **160** may be raised and lowered in order to allow suction cups **162** to engage with different nests **600** of container closures **610** contained at differing depths inside tub **630.** To this end, vacuum pickup system **160** may comprise a bellows feed-through allowing vertical motion whilst maintaining the aseptic integrity of chamber **100.** Suitable vacuum pumps, or vacuum lines from a vacuum source external to system **1000,** may be connected to vacuum pickup systems **150** and **160,** and ensure suitable vacuum at the suction cups **152** and **162.**

Cameras **210** and **220** are disposed to view and record the positioning of suction cups **152 and 162** on nests **500** and **600** respectively. In the embodiment shown in **FIG.1A****,** cameras **210** and **220** are disposed within chamber **200** and view nests **500** and **600** through sealed windows **112** and **122** respectively. In other embodiments, cameras **210** and **220** may be disposed within chamber **100** and view nests directly from within chamber **100.**

Container closing ram system **180,** shown in **FIG.1A****,** **FIG.1B****,** and **FIG.1D****,** comprises upper ram plate **182** disposed within chamber **100** above rotary stage **130,** lower ram plate **184** disposed within chamber **100** below rotary stage **130,** and ram drive **310** within chamber **300.** Ram drive **310** is disposed for driving lower ram plate **184** vertically toward upper ram plate **182** via bellows feed-through **186.** Loose base plate **138** of fiducial locating opening **136,** when located above lower ram plate **184** by suitably rotating rotary stage **130,** is pushed upward by ram plate **184** and is guided in the process by the fiducial retaining guides **137** (See **FIG.1D****).** When closures **610** in closure nest **600** are ultimately pushed against upper ram plate **182,** they are forced into the openings of containers **510** in nest **500.** This creates a sandwiched nest of closed containers **510,** each closed by a corresponding closure **610.** As shown in **FIG.1D****,** nests **500** and **600** are forced together in the process to create a compound nest **500/600.**

Controller **400,** shown in **FIG.1A** and **FIG.1B****,** may communicate with the rest of system **1000** via control communications line **410,** or may be contained physically within system **1000,** for example, within chamber **200.** Controller **400** may have suitable memory and a processor containing suitable software programming instructions which, when loaded in the memory executed by the processor, control the motions of ram system **180,** vertical motion and rotating action of rotary stage **130,** the application of vacuum to vacuum pickup systems **150** and **160,** the imaging by cameras **210** and **220,** the vertical motion of vacuum pickup system **160,** any rotational or vertical motions required from cover removal stations **140** and filling station **170,** as well as the on-and-off valving of the pharmaceutical fluid product supply to dispenser head **174.** Suitable valves and pumps, typically peristaltic pumps, required for the pharmaceutical fluid product supply to dispenser head **174** are well known in the art and may be housed in chamber **200** or may be located outside system **1000.** The various mechanical drives for the subsystems described above are well-known in the art, will not be discussed here in detail. These may typically be housed in chamber **200** of system **1000.** The software, when executed by the processor, instructs the rotary stage to rotate to angular positions that are either predetermined or based on image information from the cameras and controls the cover removal stations, the filling station, the vacuum pickup systems, and the ram system to operate specifically in conjunction with the rotary stage.

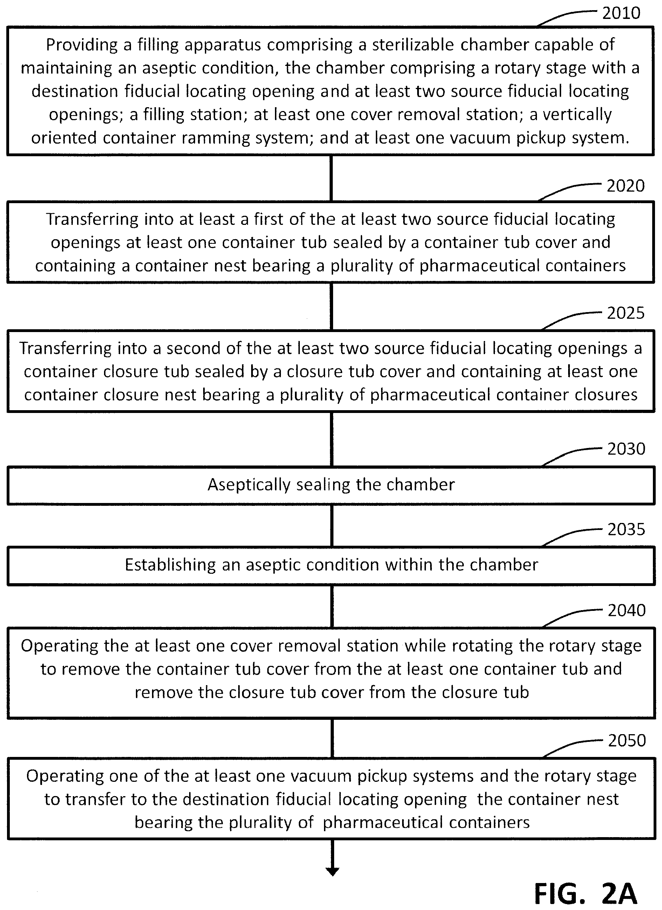

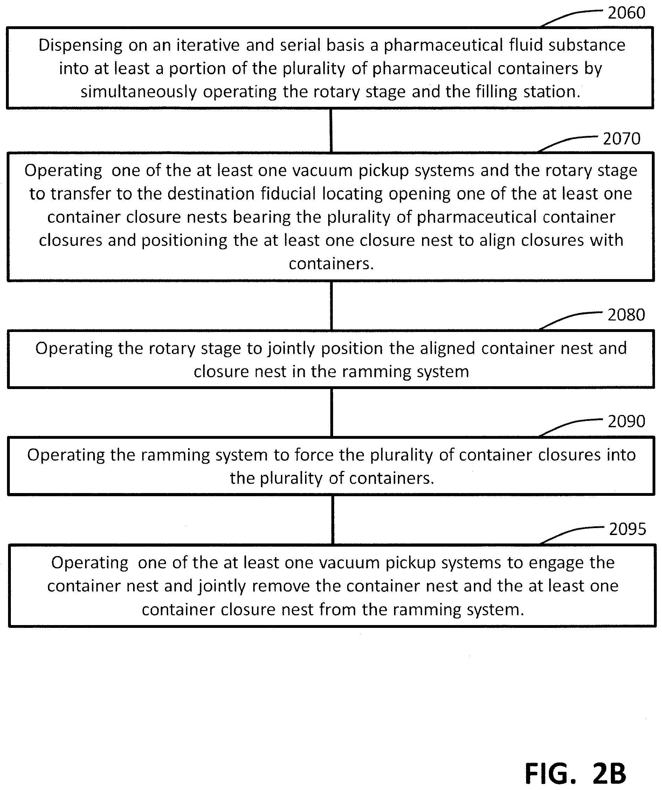

A method based on system **1000** for filling nested pharmaceutical containers with a pharmaceutical fluid product will now be described at the hand of the flow chart given in **FIG.2A****,** and which is continued in **FIG.2B****.** The method comprises providing **[2010]** filling apparatus **1000** comprising sterilizable chamber **100** capable of maintaining an aseptic condition, the chamber comprising rotary stage **130** with destination fiducial locating opening **136** and at least two source fiducial locating openings **(132** and **134);** filling station **170;** at least one cover removal station **140;** vertically oriented container ramming system **180;** and at least one vacuum pickup system (for example **150** and/or **160).** The method further comprises transferring **[2020]** into at least a first of the at least two source fiducial locating openings **(132** and **134)** at least one container tub **530** sealed by container tub cover **520** and containing container nest **500** bearing a plurality of pharmaceutical containers **510;** and transferring **[2025]** into a second of the at least two source fiducial locating openings **(134** and **132)** container closure tub **630** sealed by a closure tub cover and containing at least one container closure nest **600** bearing a plurality of pharmaceutical container closures **610.**

The method further comprises aseptically sealing **[2030]** the chamber **100** and establishing **[2035]** an aseptic condition within the chamber **100.** The establishing **[2035]** an aseptic condition within chamber **100** may comprise treating the interior of chamber **100** with any one or more of steam, hydrogen peroxide vapor, ozone, nitrogen dioxide, and ethylene oxide.

The method further comprises operating **[2040]** the at least one cover removal station **140** and rotating rotary stage **130** to remove container tub cover **520** from the at least one container tub **530** and remove closure tub cover from closure tub **630;** operating **[2050]** rotary stage **130** and one of the at least one vacuum pickup systems (for example **150** and/or **160)** to transfer to destination fiducial locating opening **136** container nest **500** bearing the plurality of pharmaceutical containers **510;** and dispensing **[2060]** on an iterative and serial basis a pharmaceutical fluid substance into at least a portion of the plurality of pharmaceutical containers **510** by operating rotary stage **130** and filling station **170.** The phrase "iterative and serial" is employed in this specification to describe the fact that the same operational steps are repeatedly used to fill the various containers and the fact that the containers are filled one after another, as opposed to simultaneously. In some embodiments multiple containers may be simultaneously filled using a filling station with multiple dispenser heads.

Steps **[2040], [2050],** and **[2060]** each involves rotating rotary stage **130** and operating another device, being respectively cover removal station **140,** one of the at least one vacuum pickup systems (for example **150** and/or **160),** and filling station **170.** The motions involved may be simultaneous in some cases or embodiments, and serial in other cases or embodiments. In some embodiments some of the motions may be simultaneous and others may be serial.

The operating **[2040]** the at least one cover removal station **140** may comprise engaging an engagement tool (for example tool **142)** with a cover removal fixture (for example fixture **540)** pre-attached to the cover being removed. Operating **[2050]** one of the at least one vacuum pickup systems may comprise contacting container nest **500** with a plurality of suction cups **152** while applying a vacuum to suction cups **152.** The dispensing **[2060]** a pharmaceutical fluid substance into at least a portion of the plurality of pharmaceutical containers may comprise disposing on an iterative and serial basis fluid product dispenser head **174** of filling station **170** over the openings of the at least a portion of the plurality of pharmaceutical containers **510.** The operating **[2050]** rotary stage **130** and one of the at least one vacuum pickup systems may comprise operating camera **210** to obtain image information of container nest **500** bearing the plurality of pharmaceutical containers **510** and to position the one of the at least one vacuum pickup systems over container nest **500.**

The method further comprises operating **[2070]** one of the at least one vacuum pickup systems (for example **150** and/or **160)** and rotary stage **130** to transfer to destination fiducial locating opening **136** one of the at least one container closure nests **600** bearing the plurality of pharmaceutical container closures **610** and positioning the at least one closure nest **600** to align closures **610** with containers **510;** operating **[2080]** rotary stage **130** to jointly position aligned container nest **500** and closure nest **600** in ramming system **180;** and operating **[2090]** ramming system **180** to force the plurality of container closures **610** into the plurality of containers **510.**

Operating **[2070]** one of the at least one vacuum pickup systems may comprise contacting container closure nest **600** with a plurality of suction cups **162** while applying a vacuum to suction cups **162.** Operating **[2090]** ramming system **180** may comprise driving the plurality of pharmaceutical containers **510** toward upper ram plate **182** of ramming system **180.**

Operating **[2070]** rotary stage **130** and one of the at least one vacuum pickup systems may comprise operating camera **220** to obtain image information of the one of the at least one container closure nests **600** bearing the plurality of pharmaceutical container closures **610** and to position the one of the at least one vacuum pickup systems over the one of the at least one container closure nests **600.**

Providing **[2010]** a filling apparatus may comprise providing a filling apparatus further comprising controller **400** and a software program executable by controller **400.** Any one or more of aseptically sealing **[2030]** chamber **100;** establishing **[2035]** an aseptic condition within chamber **100;** operating rotary stage **130;** operating the at least one cover removal station **140;** operating **[2070]** one of the at least one vacuum pickup systems **(150** and/or **160);** operating filling station **170;** and operating **[2090]** ramming system **180** may be done automatically by executing the software program in the controller.

In the embodiment described at the hand of Figures **1A** to **1F****,** each of steps **[2040], [2050], [2060], [2070],** and **[2080]** comprises rotating a rotary stage, for example rotary stage **130,** bearing the container nests and container closure nests.

In other embodiments a plurality of the steps of removing a container tub cover from at least one container tub **530;** removing a container tub cover from at least one container closure tub **630;** transferring to destination fiducial locating opening **136** container nest **500;** dispensing a pharmaceutical fluid substance into pharmaceutical containers **510;** transferring to destination fiducial locating opening **136** one of the at least one container closure nests **600;** and positioning aligned container nest **500** and closure nest **600** in ramming system **180** comprises rotating a rotary stage bearing the container nests and container closure nests.

In a general embodiment, at least one of the steps of removing a container tub cover from at least one container tub **530;** removing a container tub cover from at least one container closure tub **630;** transferring to destination fiducial locating opening **136** container nest **500;** dispensing a pharmaceutical fluid substance into pharmaceutical containers **510;** transferring to destination fiducial locating opening **136** one of the at least one container closure nests **600;** and positioning aligned container nest **500** and closure nest **600** in ramming system **180** comprises rotating a rotary stage bearing the container nests and container closure nests.

It is to be noted that neither filling system **1000,** nor the associated method, needs to employ the vibratory bowls or escapements that are typical of the prior art. Unlike many prior art systems, filling system **1000** also does not require the use of gloves for use by the operator to access the interior of the chamber.

The system above has been described as employing a controller that runs stored software running on a general-purpose computer platform, but it may also be implemented in whole or in part using special-purpose hardware.

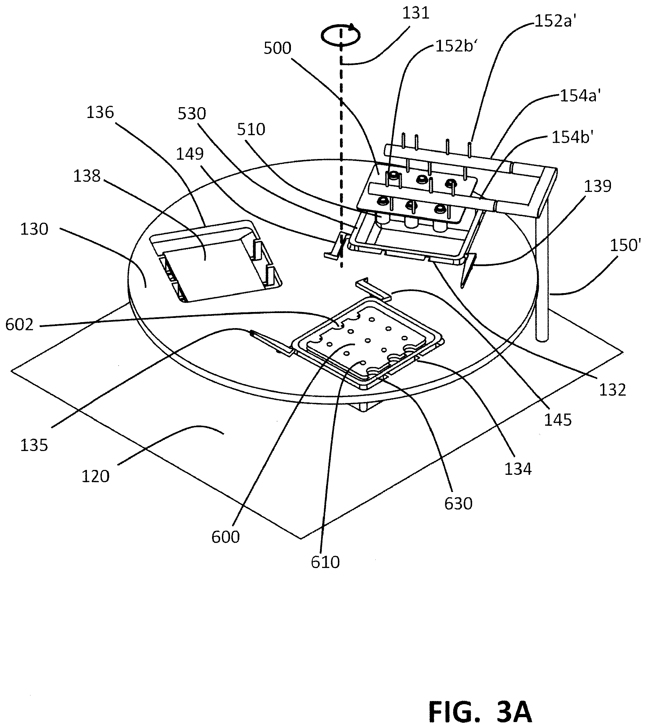

The system described above also employs fiducial openings defined in the rotary stage to hold the tubs and nests, but it may also employ other types of fiducial structures that include other configurations of constraining surfaces sufficient to hold the tubs and nests in place. Notched posts mounted on the rotary stage may hold the tubs and/or nests above the rotary stage, for example. Further fiducial locating structures for holding tubs of nests for containers or container closures will be described below at the hand of **FIGS.3A****,** **3B****,** **4A****,** and **5A****.**

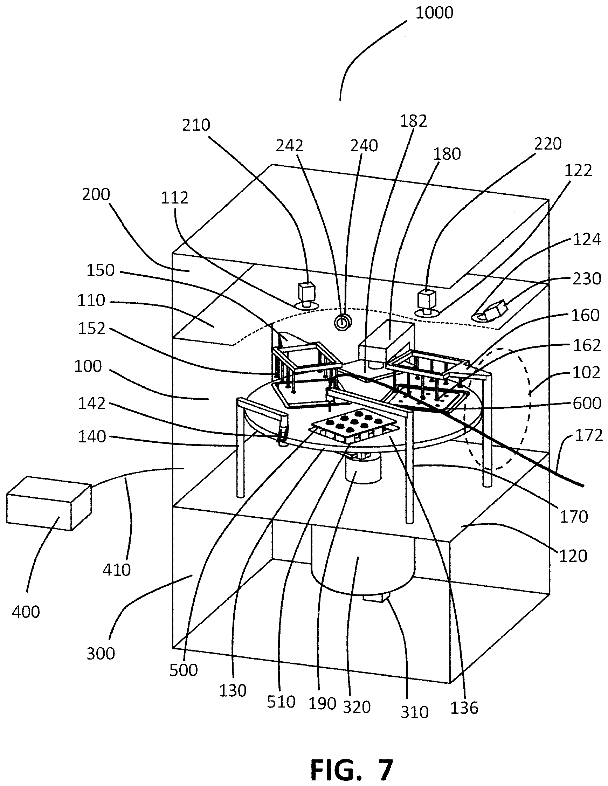

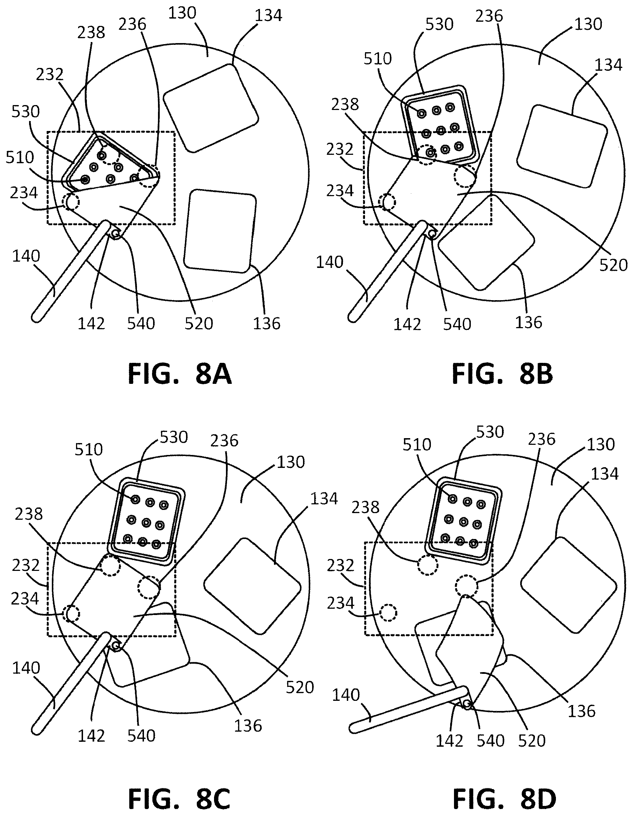

As shown in **FIG.7** and **FIG.8A-D,**filling system **1000** may comprise peeling monitor sensor **230** disposed to collect light from a source fiducial locating opening, for example **132** or **134,** when fiducial locating opening **132** or **134** is located proximate cover removal station **140** to allow the removal of a cover from a tub located in source fiducial locating opening **132** or **134.** Peeling monitor sensor **230** may be located behind sealed window **124** separating sensor **230** from sealable chamber **100.** Peeling monitor sensor **230** may be a CCD camera or any other light sensor that is addressable or configurable via software or other suitable means to provide a total light signal from a pre-defined portion of its field of view.

In **FIG.8A-D,**container tub **530** is shown located in fiducial locating opening **132,** which is obscured by tub **530** while tub **530** is having its cover **520** removed or peeled by cover removal station **140.** The use of engagement tool **142** and cover removal fixture **540** attached to cover **520** has already been described.

As shown in **FIG.7****,** filling system **1000** may comprise peeling monitor light source **240** disposed to illuminate a portion of rotary stage **130** proximate cover removal station **140.** Peeling monitor light source **240** provides a suitably high amount of illumination to ensure that its illumination dominates strongly over any ambient light in system **1000.** In order to further ensure that the illumination from light source **240** may be differentiated from ambient light, light source **240** may be of a selected wavelength or wavelength range. One suitable choice of device for light source **240** may be an infrared light emitting diode. Light source **240** may be provided with optical wavelength filter **242** that transmits only light of suitably differentiated infrared radiation. Light source **240** may illuminate the relevant portion of rotary stage **130** through sealed window separating light source **240** from sealable chamber **100.** For the sake of clarity, sealed window separating light source **240** from sealable chamber **100** is not shown in **FIG.7****.**

Since suitable contrast is advantageous in monitoring the cover removal or the "peeling" process, and since rotary stage **130** may have a surface exhibiting an amount of reflectivity that may interfere with the working of peeling monitor sensor **230,** light source **240** and peeling monitor sensor **230** maybe disposed relative to each other in such fashion as to ensure that light from light source **240** that is directly reflected by rotary stage **130** is not directed toward peeling monitor sensor **230.** To this end, peeling monitor sensor **230** may be disposed facing the portion of rotary stage **130** proximate cover removal station **140,** but located at a position that is outside any plane defined by any line perpendicular to rotary stage **130** within the area of rotary stage **130** illuminated by source **240** and the path of any light from light source **240** to the point where the perpendicular line intersects the illuminated surface of rotary stage **130.** However, it is also advantageous for peeling monitor sensor **230** to have as near to a plan view of the illuminated area of rotary stage **130** as possible, without suffering direct reflection from rotary stage **130.**

In **FIG.7****,** an example placement of peeling monitor sensor **230** and light source **240** is given. The portion of rotary stage **130** proximate cover removal station **140** is illuminated by light source **240** at a slanting angle, with light source **240** disposed in the back wall of sealable chamber **100** in order to achieve the required angle. Peeling monitor sensor **230** is disposed in upper wall **110** of sealable chamber **100.** As is readily evident from **FIG.7****,** light from light source **240** will not be specularly reflected by the surface of the portion of rotary stage **130** proximate cover removal station **140.** This allows light from light source **240** diffusely reflected by cover **520** of tub **530,** when being removed from tub **530** by cover removal station **140,** to dominate in the light received by peeling monitor sensor **230.**

We turn now to **FIG.8A-D,**representing a progression of stages in the removal of cover **520** from tub **530** by cover removal station **140.** In the present specification, the phrase "monitored area" is used to describe area **232** of **FIG.8A-D,**that is illuminated by light source **240** and monitored by peeling monitor sensor **230.** During the peeling or removal of cover **520** in system **1000,** rotary stage **130** is rotated and therefore monitored area **232** changes position on rotary stage **130** in the process.

Other pharmaceutical filling systems may be arranged differently and may function differently as regards the location of fiducial locating openings for tubs. For example, in such other systems the fiducial locating openings may be stationary and the covers may be removed solely by manipulating an associated cover removal station. In yet further systems, the fiducial locating openings may be moved linearly, rather than being rotated as in the case of system **1000.** In the broadest implementation of the monitoring and control system for cover removal from tubs bearing pharmaceutical containers or closures, we concern ourselves only with the fact that the cover is somehow peeled from the tub it is attached to.

In **FIG.8A-D,**tub **530** has a substantially rectangular shape with four corners and entire tub **530** is tightly sealed against the environment by cover **520** when tub **530** is initially placed in fiducial locating opening **132,** which is obscured by tub **530** in **FIG.8A-D,**Cover **520** has four corresponding corners. The first corner of cover **520** to be peeled from tub **530** is the corner that is shown furthest from cover removal station **140** in **FIG.8A****.** In one implementation of the cover removal monitoring and control system of the present specification the peeling of the first corner of the cover is not monitored.

**FIG.8A** shows the situation very shortly after the peeling of the next corner of cover **520,** being in this embodiment the corner of cover **520** corresponding to the leftmost corner of tub **530 in** **FIG.8A****.** In the case of system **1000,** the peeling of the first and second corners of cover **520** is by rotation of rotary stage **130** while the first corner of cover **520** is held by cover removal station **140** using engagement tool **142** and cover removal fixture **540,** as described above in the present specification.

By suitable choice of the exact location of a first peeling monitor zone **234** within monitored area **232,** proximate the second corner of tub **530** and substantially non-overlapping with container tub **530** immediately upon completion of the peeling of cover **520** from the second corner of tub **530,** the light signal or image brightness produced by sensor **230** when the second corner of cover **520** has been peeled may be made to be very large, allowing thereby a sensitive measure of whether or not the second corner has been successfully peeled. This results from generally white cover **520** substantially covering monitor zone **234** at the moment of peeling being completed and diffusely reflecting light from light source **240** in the direction of peeling monitor sensor **230.**

The term "peeling monitor zone" is used in this specification to describe a range of positions that the system is configured to evaluate in determining peeling status. This may be an entire three-dimensional volume having a base defined by, for example, dotted line **232** in **FIG.8A** and an apex defined by sensor **230,** making peeling monitor zone **234** an oblique cone. To the extent that cover **520,** upon being peeled, intrudes into this oblique cone, cover **520** is overlapping into peeling monitor zone **230.** A variety of other suitable zone dimensions may also be provided, such as a collimated cylindrical zone, a narrowly focused beam, or even a moving scanned beam. It is also to be noted that, in general, the base of the three-dimensional volume constituting the peeling monitor zone does not have to be restricted to a single plane, nor does it have to have a base that is a conic section. The base may be a defined portion of a field of view of sensor **230,** and may be distributed over different physical surfaces within filling system **1000.** It may also be possible to restrict how far the detection zone extends above the base.

The capture of the signal or image from sensor **230** may be timed to coincide with the moment that peeling of the second corner is completed. If sensor **230** is an imaging sensor, monitor zone **234** may be software-selected within the image produced by sensor **230.** If sensor **230** is non-imaging sensor, then a simple measurement of absolute light signal will serve the same purpose. In both implementations, the distinction between unpeeled and peeled may be made on the basis of the total light signal received from first corner peeling monitor zone **234.** To differentiate a good peel from a bad peel, a "floor value" may be set for the absolute signal measured by sensor **230** from monitor zone **234.** If sensor **230** is an imaging sensor, then the measurements may be, for example without limitation, an average over the image received from monitor zone **234.** It is to be noted that the method requires no extensive image management or perspective correction, nor any compensation for lens distortions and the like, as are often required in machine vision applications. And while it is presently contemplated that the method would be performed in connection with software running on controller **400** or other processor, it may be implemented at least in part with dedicated hardware.

In **FIG.8B** the peeling of a third corner of cover **520** is shown, being the right-most corner of cover **520** in **FIG.8A****.** In **FIG.8B****,** rotary stage **130** has been rotated a number of degrees clockwise with respect to **FIG.8A** so as to peel the third corner of cover **520,** which is shown in **FIG.8B** as having been peeled. As is the case with the second corner of cover **520,** by suitable choice of the exact location of peeling monitor zone **236,** proximate the third corner of tub **530** and substantially non-overlapping with container tub **530** immediately upon completion of the peeling of cover **520** from the third corner of tub **530,** the diffusely reflected signal or image produced by sensor **230** when the third corner of cover **520** has been peeled may be caused to be very large, allowing thereby a sensitive measure of whether or not the third corner has been successfully peeled. The method for determining the quality of the peeling of the third corner may proceed exactly as is the case with the second corner, with the exception that it is light reflected from peeling monitor zone **236** that is used to make the determination. In the case of system **1000,** the peeling of the first, second, and third corners of cover **520** is by rotation of rotary stage **130** while the first corner of cover **520** is held by cover removal station **140** using engagement tool **142** and cover removal fixture **540,** as described above in the present specification.

The peeling of the fourth and last corner of cover **520,** as per **FIGS.8C** and **D,** presents somewhat of a different challenge. Unlike the second and third corners, the quality of the peel cannot be assessed solely on the basis of the white diffusely reflecting cover **520** covering any peeling monitor zone proximate tub **530,** because that would actually be evidence of the peeling having not been completed. A slightly different approach is therefore followed. Again a suitable peeling monitor zone is chosen, being zone **238** proximate the fourth corner of tub **530** and substantially non-overlapping with tub **530** immediately upon completion of the peeling of cover **520** from the fourth corner of tub **530.** Two measurements are made of zone **238,** namely, a measurement at the time of **FIG.8C** just before cover **520** finally detaches from tub **530,** and another as per **FIG.8D** after it has been detached by the rotation of cover removal station **140.** The first of the two measurements should have a high signal as a result of cover **540** diffusely reflecting a lot of light from light source **240** in the direction of peeling monitor sensor **230,** and the second measurement should be very low, due to the absence of any white diffusely reflecting cover material of cover **520** in zone **238.** In a general implementation of the monitoring and control system for cover removal from tubs bearing pharmaceutical containers or closures, rotary stage **130** and cover removal station **140** may be moved sequentially or may be moved simultaneously.

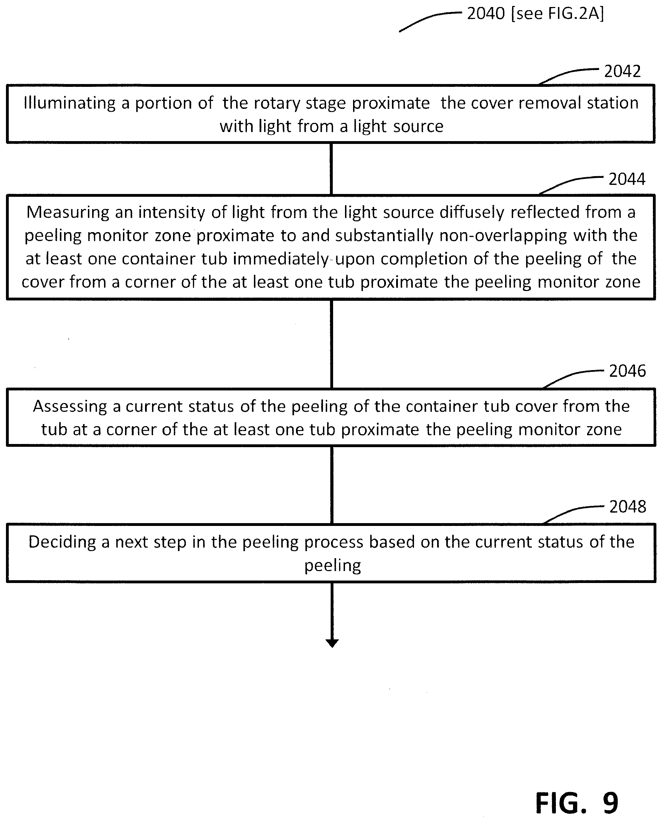

Based on the above description, the method for aseptically filling pharmaceutical containers with a pharmaceutical fluid substance described above at the hand of the flow chart of **FIG. 2A** may further comprise as per the flow chart in **FIG.9****:** monitoring and controlling removing **[2040]** container tub cover **520** from the at least one container tub **530** by a method comprising: illuminating **[2042]** portion **232** of rotary stage **130** proximate cover removal station **140** with light from light source **240;** measuring **[2044]** an intensity of light from light source **240** diffusely reflected from peeling monitor zone **234, 236, 238** proximate to and substantially non-overlapping with the at least one container tub **530** immediately upon completion of the peeling of cover **520** from a corner of the at least one tub **530** proximate peeling monitor zone **234, 236, 238;** assessing **[2046]** a current status of the peeling of container tub cover **520** from the at least one tub **530** at a corner of tub **530** proximate peeling monitor zone **234, 236, 238;** deciding **[2048]** a next step in the peeling process based on the current status of the peeling.

In the above description of the peeling process, the peeling monitor zones have been chosen to be proximate the relevant corner being peeled and substantially non-overlapping with tub **530** and the measurements of reflected light are done immediately upon completion of the peeling of cover **520** from the relevant corners of tub **530.** It will be understood that in some embodiments the measurements may be done at predetermined times after completion of the peelings of the corresponding corners, and the corresponding peeling monitor zones may be chosen to be at commensurately different corresponding locations proximate the corresponding corners of tub **530** and substantially non-overlapping with tub **530,** and still in the illuminated portion of stage **130.**

In some embodiments, instead of single peeling monitor sensor **230** being employed, a plurality of separate peeling monitor sensors may be employed with a different one of the plurality of sensors dedicated to measuring the light reflected from each individual peeling monitor zone, for example peeling monitor zones **234, 236,** and **238.** In further embodiments, a plurality of light sources may be employed, allowing a separate light source to serve as illumination for each corresponding corner of tub **530** to be monitored for peeling. In yet further embodiments, different wavelengths of illumination may be employed for the different corners to be monitored and the sources and sensors matched accordingly by wavelength or wavelength range.

The output of sensor **230** may be used in a variety of ways. In one embodiment, the sensor may provide via software in controller **400** a signal that indicates whether a corner has been successfully peeled. If this signal indicates successful peeling of one corner, the controller may cause the system to continue peeling the cover until the next sensing step is performed, and this process may continue until the cover is completely removed. If the sensor detects a failure to remove a corner, the controller may stop the movement of the platform, issue an alert, and/or provide another action that will allow the condition to be inspected and/or remedied.

Assessing **[2045]** may comprise comparing the intensity of light from measuring **[2044]** with a predetermined light intensity value. The predetermined light intensity value may be chosen such that, if it is exceeded by the measured light intensity during measuring **[2044],** it represents a large presence of tub cover **520** within peeling monitor zone **234, 236, 238,** which in turn is evidence of tub cover **520** having been successfully removed from the corner of tub **530** proximate peeling monitor zone **234, 236, 238.** Illuminating **[2042]** with light from light source **240** may be illuminating **[2042]** with infrared light from light source **240.**

The predetermined light intensity value may be stored in the memory of controller **400.** Controller **400** may contain suitable software programming instructions which, when loaded in the memory and executed by the processor, control the rotating action of rotary stage **130,** measurements by sensor **230,** and any rotational or vertical motions required from cover removal station **140** in mutually dependent and synchronized fashion. This allows measuring **[2044]** to be timed to take place immediately upon completion of the peeling of the cover **520** from a corner of the at least one tub **530** proximate peeling monitor zone **234, 236, 238.** The second measurement associated with the fourth corner of tub **530** may similarly be timed to take place when tub cover **520** has been completely detached from tub **530.** An assessment of the peeling of the last corner of tub **530** is made based on the last measurement returning a light intensity that is lower than a predetermined value associated with peeling monitor zone **238.** The sequence of high diffusely reflected light intensity followed by low diffusely reflected light intensity is evidence of the peeling at the last corner respectively taking place and cover **520** having been completely detached and removed from peeling monitor zone **238.** By the above approach the cover removal monitoring and control process may be completely automated.

The same cover removal station **140,** light source **240,** sensor **230,** and peeling monitor zones **234, 236, 238** may be employed to also remove a cover from a container closure tub by the same method as described above.

Though the apparatus and method for monitoring the removal of a cover from a tub has been described above at the hand of a system employing a rotary turntable comprising a simple fiducial locating opening for holding the tub in a predetermined fixed position, this represents but one non-limiting example of a suitable system comprising a platform having a fiducial source locating structure. In general, the platform is not limited to being a rotary stage. The platform may be a movable platform and may be movable through any general path that allows removal of the cover by a suitable cover removal station. The source fiducial locating structure may be any fiducial locating structure that is capable of holding the tub in a predetermined fixed position, including those described at the hand of **FIGS.3A****,** **3B****,** **4A****,** and **5A****.**

In one example, the system may be adapted to monitor cover removal in an apparatus of the type described in above-referenced United States Patent Application Publications US 2017-0248626 and US 2016-0251206, which employs an articulated arm to hold the tub during cover removal. Since there is no rotary stage **130** in this case, monitored zone **232** will extend over another peeling monitor surface inside the aseptic chamber, such as a top surface of a pedestal. And while it is presently preferred that the surface be a horizontal surface that is monitored from the top, it may also be possible in some instances to monitor the cover removal process from another vantage point, such as from the bottom up. The optical properties of the monitored zone or at least peeling monitor zones **234, 236, 238** may be provided with suitable optical properties to enhance the monitoring process. This may be achieved in a variety of ways, such as by the selection of the material that defines the monitored zone or by applying a coating or other surface treatment to some or all of the monitored zone.

The system may also be reorganized in a variety of ways. Instead of the top-down reflective measurement presented above, for example, a bottom-up transmisssve measurement may be performed by placing one or more light sources at one or more peeling detection zones in the in the monitored zone, such as by embedding them in the platform. The system would then detect successful peeling by looking for the cover to block light from the light sources from reaching the sensor.

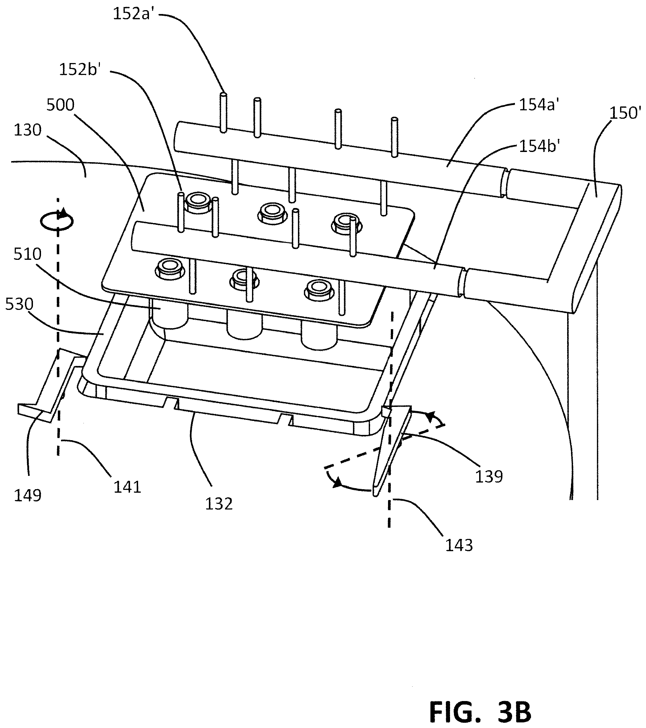

Another embodiment of a filling system according to the invention may be in all respects identical to the embodiments described above at the hand of Figures **1A** and **1B****,** with the exception of vacuum pickup system(s) **150** or **160.** Figures **3A** and **3B** show a portion of a filling system as described above. **FIG.3B****,** in particular, focuses on the general area of one of the vacuum pickup systems, by way of example, vacuum pickup system **150.** In this alternative embodiment, vacuum pickup system **150** is replaced by reconfigurable vacuum pickup system **150'.** Vacuum pickup system **160** of Figures **1A** and **1B** may similarly be replaced by reconfigurable vacuum pickup system **160'** of the same arrangement as vacuum pickup system **150'.** In the interest of clarity, vacuum pickup system **160'** is not shown in **FIG.3A** or **3B****.** In other embodiments, a single reconfigurable vacuum pickup system **150'** may be employed to pick up both container nests and container closure nests. Vacuum pickup system **150'** may access the container nests and container closure nests by rotation of rotary stage **130.**

Vacuum pickup system **150'** comprises two rotary arms **154a'** and **154b',** in their turn respectively comprising pluralities of suction cups **152a'** and **152b'.** Vacuum pickup system **150'** is arranged to pick up nests **500** of containers **510** by means of suction cups **152a'** and **152b'.** Vacuum pickup system **150'** may also be arranged to pick up nests **600** of container closures **610** by means of suction cups **152a'** and **152b'.** As with vacuum pickup system **150,** vacuum pickup system **150'** may be raised and lowered in order to allow suction cups **152a'** and **152b'** to engage with different nests **600** of container closures **610** contained at differing depths inside tub **630.**

Suction cups **152a'** and **152b'** are arranged on rotary arms **154a'** and **154b'** as pluralities of sets of linearly arranged suction cups **152a'** and **152b',** each set of linearly arranged suction cups **152a'** and **152b'** being arranged at a different angle perpendicular to the longitudinal axes of rotary arms **154a'** and **154b'.** This arrangement allows rotary arms **154a'** and **154b'** to be rotated about their longitudinal axes in order to orient different sets of linearly arranged suction cups **152a'** and **152b'** to engage with different nests **500** of containers **510.** This allows the sets of suction cups **152a'** and **152b'** to be individually selectable for use. Rotation of rotary arms **154a'** and **154b'** may be performed manually. In other embodiments, rotation of rotary arms **154a'** and **154b'** may be by means of a suitable motorized drive incorporated in vacuum pickup system **150'** and controlled by controller **400** shown in **FIG.1A****.**

By selecting different sets of linearly arranged suction cups **152a'** and **152b'** via the rotation of rotary arms **154a'** and **154b',** the sets of suction cups **152a'** and **152b'** may be disposed to engage with different container nests **500** bearing containers **510,** or container closure nests **600** bearing container closures **610.**

Figures **3A** and **3B** show vacuum pickup system **150'** as comprising two rotary arms, being rotary arms **154a'** and **154b'.** In other embodiments, one or more arms may be employed, all embodiments sharing the concept of a selectable configuration of suction cups. Whereas the selection of suction cup configurations in **FIG. 3A** and **FIG. 3B** is by means of rotation of the arms **154a'** and **154b'** bearing the suction cups **152a'** and **152b',** the selecting in other embodiments may be on a different basis of configuration, including, for example without limitation, lateral translation of suction-cup-bearing arms in a plane parallel to the rotation plane of rotary stage **130** in order to engage different sets of suction cups with container nests or container closure nests. In Figures **3A** and **3B** suction cups are arranged in linear sets. In other embodiments non-linear arrangements of suction cups may be employed.

Turning now to **FIG. 3B** specifically, we consider members **149** and **139** in more detail. In one embodiment, reconfigurable stopping member **149** is shown as having two different ends of which a first end may be selected for use by suitable rotation of reconfigurable stopping member **149** about stopping member rotation axis **141** to a predetermined set position. In the set position, reconfigurable stopping member **149** provides a hard stop for a proximal end of container **530** against the selected end of reconfigurable stopping member **149** along a direction parallel to the longitudinal axes of rotary arms **154a'** and **154b'.** In this embodiment, reconfigurable stopping member **149** may be rotated through 180 degrees to dispose the second end of reconfigurable stopping member **149** to stop container **530.** The second end of reconfigurable stopping member **149** may be configured to stop the proximal end of container **530** at a different point than where the first end of reconfigurable stopping member **149** stops the proximal end of container **530.**

Restraining member **139** is configured to push against a distal end of container **530.** While different mechanisms are contemplated to ensure the pushing action of restraining member **139,** one particular suitable means is by providing restraining member **139** with suitable spring loading to rotate about axis **143.** By the above operation, reconfigurable stopping member **149** and restraining member **139** together allow container **530** to be positioned at an exact location parallel to the longitudinal axes of rotary arms **154a'** and **154b'.** The particular exact location is selectable by selecting the appropriate end of reconfigurable stopping member **149** to stop container **530.** This arrangement allows containers **530** of different dimensions parallel to the longitudinal axes of rotary arms **154a'** and **154b'** to be located at exact predetermined locations with respect to sets of suction cups **152a'** and **152b'.**

A particular set of suction cups **152a'** and **152b'** may be selected to match the selection of the particular end of reconfigurable stopping member **149.** In this way, vacuum pickup system **150'** may be set to a configuration that ensures that a selected size of container **530** is precisely positioned to allow container nests **500** within container **530** to be engaged by specific sets of suction cups **152a'** and **152b'.** Vacuum pickup system **150'** is thereby reconfigurable to engage with nests of different sizes within containers of different sizes.

In the interest of clarity, the description above, as well as Figures **3A** and **3B****,** show an arrangement that allows for the exact positioning of containers **530** along only one dimension in the rotation plane of rotary stage **130,** the dimension of the containers perpendicular to the one dimension being assumed to be identical. In such an arrangement, fiducial locating openings **132** and **134** are sized to constrain containers **530** in the perpendicular dimension in the rotation plane of rotary stage **130.**

In another embodiment, a further reconfigurable stopping member and restraining member may be added to the arrangement of **FIG. 3A** and **FIG. 3B** in order to address the positioning of container **530** in the perpendicular direction within the rotation plane of rotary stage **130.** To allow the positioning of container **530** in this perpendicular direction, fiducial locating openings **132** and **134** are not sized to constrain containers in any direction within the rotation plane of rotary stage **130.**

In the embodiments described above, reconfigurable stopping member **149** has been described as having two ends of which one is selected for use at any one time by rotating reconfigurable stopping member **149** about stopping member rotation axis **141.** In other embodiments, reconfigurable stopping member **149** may be shaped or configured to have more than two stopping ends, the ends being selectable by suitable rotation of reconfigurable stopping member **149** about stopping member rotation axis **141.** In one embodiment, in which the reconfigurable stopping member has a very large number of stopping ends, the reconfigurable stopping member may assume the shape of a cam, representing a large plurality of possible stopping ends that may be selected via rotation of the reconfigurable stopping member about a suitable stopping member rotation axis.

In general, the system described at the hand of Figures **3A** and **3B** comprises a reconfigurable fiducial nest positioning system. The reconfigurable fiducial nest positioning system comprises a movable platform comprising fiducial locating opening **132,** reconfigurable stopping member **149,** and restraining member **139.** In the case of the system of Figures **3A** and **3B****,** the movable platform is rotary stage **130.** As explained later, other movable platforms are also contemplated. To the extent that, for example, tub **530** positionally constrains and locates nest **500** inside tub **530,** any system that fiducially locates tub **530** inherently also fiducially locates nest **500.**

The various embodiments contemplated all comprise a reconfigurable vacuum pickup system that may be configured to engage its suction cups with corresponding areas on a pharmaceutical container nest. The containers in the container nest may be closed by corresponding container closures suspended in a container closure nest. The planar surface of the container closure nest may have an outline that leaves pass-throughs on its perimeter for the suction cups to pass through to engage with the container nest. By way of example, in **FIG.3a** pass-throughs **602** are shown on the perimeter of closure nest **600.** Alternatively or additionally, the container closure nest may have suitable openings in its planar interior to serve as pass-throughs for the suction cups to pass through to engage with the container nest. The vacuum pickup systems contemplated are further configured and disposed to pick up the combination of nested containers and their closures by the container nest, as opposed to by the closure nest.

In a general embodiment, a nest handling subsystem comprises a reconfigurable vacuum pickup system for picking up container nests and/or container closure nests may comprise one or more arms bearing a plurality of sets of suction cups. By reconfiguration of the vacuum pickup system a set of suction cups may be selected from among the plurality of sets of suction cups, the selected set of suction cups being pre-arranged to engage with a particular container nest or container closure nest. The selection may be on the basis of one or both of the size and the shape of the nest. The nest handling system may further comprise at least one pair of reconfigurable stopping members **149** and restraining member **139** disposed proximate opposing ends of fiducial locating opening **132** for holding tub **530** containing container nests **500** bearing containers **510** in order to engage with opposing ends of tub **530.** The stopping and restraining members are disposed to position tub **530** in a predetermined position that ensures that the selected set of suction cups may engage with the container nests and/or container closure nests.

As is the case with opening **132,** opening **134** of **FIG.3A** may also be served by at least one set of a reconfigurable stopping member, being member **145** in this case, and a restraining member, being member **135** in this case. Reconfigurable stopping member **145** and restraining member **135** function with respect any tub in opening **134** in the same way as reconfigurable stopping member **149** and restraining member **139** function with respect any tub in opening **132.**

The various embodiments above have been described in terms of **FIG.1A** to **E** and **FIG.3A****,** and **FIG.3B** in which the vacuum pickup system **150, 160** is described as part of pharmaceutical filling system **1000.** However, vacuum pickup system **150', 160'** may also be employed in its own right other apparatus not limited to the filling system of **FIG.1A** to **1E**, or, in fact, to filling systems in general. Some other example applications include, without limitation, lyophilizing systems. It may be applied to suitable nests of any objects arranged in a predetermined pattern. Furthermore, while the system **1000** of **FIG.1A** to **FIG.1E** employs rotary stage **130,** reconfigurable vacuum pickup system **150'** may employ any suitable movable platform comprising suitable fiducial locating openings.

The method described above at the hand of **FIG. 2A** and **2B** may now also be described in more detail with reference to **FIG. 3A** and **FIG.3B****.** The providing at least one vacuum pickup system as part of the providing a filling apparatus step **[2010]** may comprise providing at least one reconfigurable vacuum pickup system **150',** the at least one reconfigurable vacuum pickup system **150'** comprising a plurality of sets of suction cups **152a'** and **152b'.**

The providing a filling apparatus step **[2010]** may comprise providing rotary stage **130** with destination fiducial locating opening **136** and at least two source fiducial locating openings **132, 134,** each source fiducial opening having at least one pair of reconfigurable stopping members **149** and a restraining member **139.**

The transferring step **[2020]** may comprise operating at least first reconfigurable stopping member **149** to stop container tub **530** at a predetermined container tub position and operating at least first restraining member **139** to restrain container tub **530** at the predetermined container tub position.

The transferring step **[2025]** may comprise operating at least second reconfigurable stopping member **145** to stop container closure tub **630** at a predetermined closure tub position and operating at least second restraining member **135** to restrain container tub **630** at the predetermined closure tub position.

The step of operating **[2050]** the at least one vacuum pickup system **150', 160'** may comprise configuring the at least one reconfigurable vacuum pickup system **150', 160'** to select a first predetermined set of suction cups disposed to engage with container nest **500.**

The operating **[2070]** of one of the at least one vacuum pickup system **150', 160'** may comprise configuring the at least one reconfigurable vacuum pickup system **150', 160'** to select a second predetermined set of suction cups disposed for engaging with container closure nest **600.**

The method may further comprise operating **[2095]** at least one vacuum pickup system **150', 160'** with the first predetermined set of suction cups selected to engage with container nest **500** and jointly remove container nest **500** and container closure nest **600** from ramming system **180.**

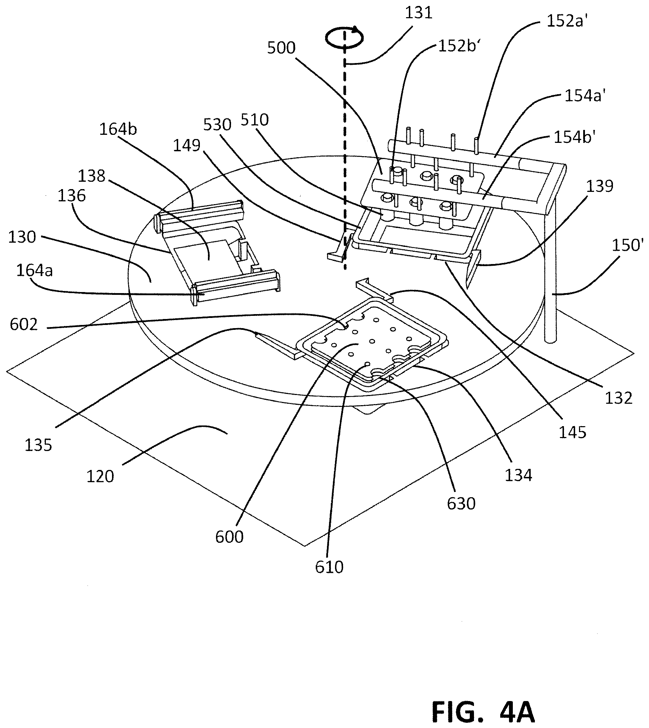

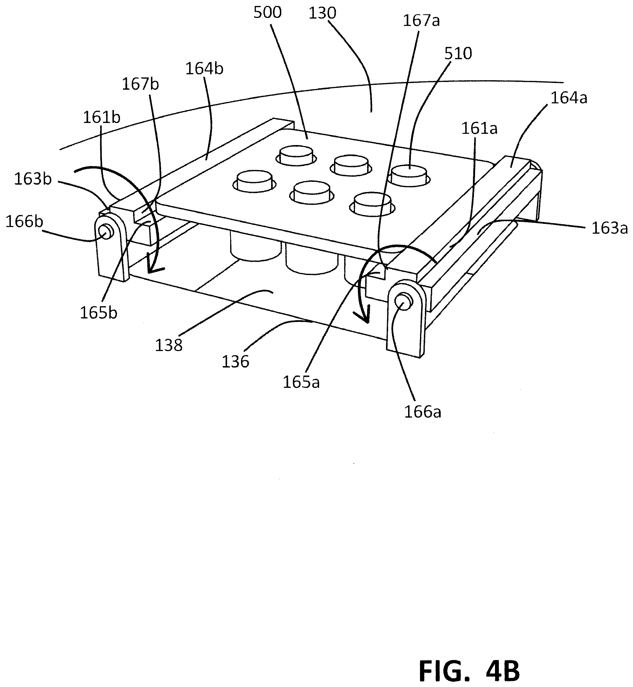

We have considered in **FIG 3A** and **FIG.3B** alternative embodiments of the arrangements of vacuum pickup systems **150** and **160** of **FIG. 1a** in the form of vacuum pickup systems **150'** and **160';** and the positioning arrangements associated with source openings **132** and **134** in the form of elements **135,145,139,** and **149.** We now turn our attention to alternative embodiments for the arrangements around destination opening **136** of **FIG 1A** and **FIG.3A****.** **FIG 4A** and its close-up view in **FIG.4B** show the system of **FIG.3A** with a different embodiment of the arrangement around destination opening **136.** While cameras **210** and **220** of **FIG.1A** may be employed in conjunction with controller **400** and rotation of rotary stage **130** to position nest **500** at opening **136,** and to position nest **600** over nest **500** at opening **136,** the adjustable destination fiducial positioning system of **FIG.4A** and **FIG.4B** comprising rotary positioning elements **164a** and **164b** may be alternatively or additionally employed to accurately position nests **600** and **500.**

Typical industrial container nests are not manufactured to a dimensional standard, and, as a result, any system for filling and closing nested containers **510** has to have a mechanism to accurately position differently sized nests **500** bearing containers **510.** To this end, rotary positioning elements **164a** and **164b** may have different sets of paired positioning surfaces **167a,167b** and **163a,163b** allowing nests **500** of specific dimensions to be accurately fitted between such paired positioning surfaces. In **Fig.4B****,** nest **500** fits such that its two opposing ends in a first dimension touch mutually facing surfaces **167a** and **167b** of rotary positioning elements **164a** and **164b** respectively. By mutually counter-rotating elements **164a** and **164b** about respectively axes **166a** and **166b,** surfaces **167a** and **167b** may be made to face each other and may thereby allow the precise positioning between them of a nest of different length in the first dimension.

As is evident from **FIG.4B****,** when surfaces **167a** and **167b** face each other, the nest positioned snugly between them may be retained in a precise and predetermined vertical position by resting on surfaces **165a** and **165b** of rotary positioning elements **164a** and **164b** respectively. When surfaces **163a** and **163b** face each other, the alternative nest positioned snugly between them may retained in a precise and predetermined vertical position by resting on surfaces **161a** and **161b** of rotary positioning elements **164a** and **164b** respectively. Elements **164a** and **164b** may be rotated manually about axes **166a** and **166b** respectively. In some embodiments, the rotation of elements **164a** and **164b** may be done automatically by means of motorized drives controlled by controller **400** and suitable control software. That control may be based on predetermined dimensional data relating to the nest being positioned between the surfaces of elements **164a** and **164b.** It may also be based on input data derived from imaging data obtained from cameras **210** and/or **220.** Further, the rotation may take place as nest **500** is lowered into position so that the particular surfaces of elements **164a** and **164b** destined to engage with the opposing ends of nest **500** along the first dimension may serve as closing horizontal grip on nest **500** as the surfaces rotate toward the position in which they face each other. In this embodiment, the horizontal positioning and vertical positioning of a nest between elements **164a** and **164b** are not mutually independent.

Another arrangement as shown in **FIG.4A** and **FIG.4B** for the first dimension of nest **500,** may also be established for the second planar dimension of nest **500** perpendicular to the first dimension. This allows any nest **500** placed at opening 136 to be accurately located in a location predetermined by the choice of setting of rotary positioning elements **164a** and **164b.**

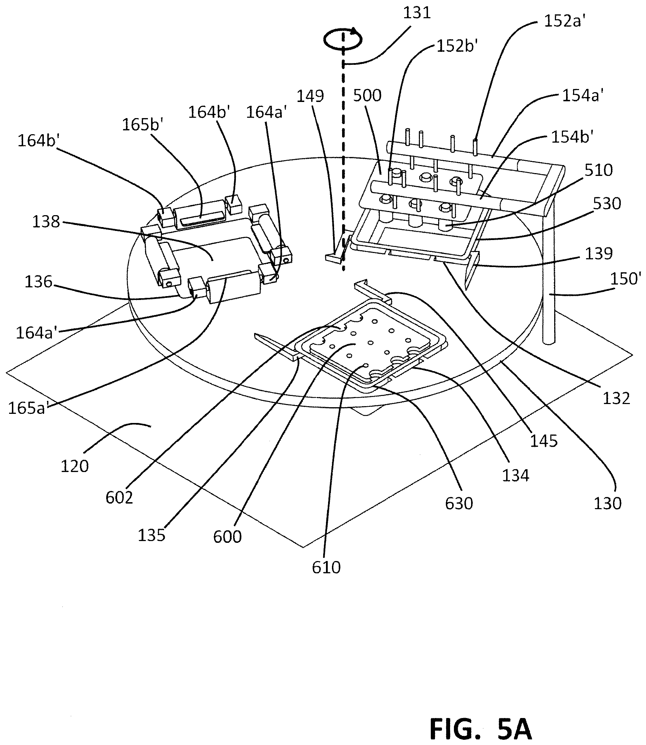

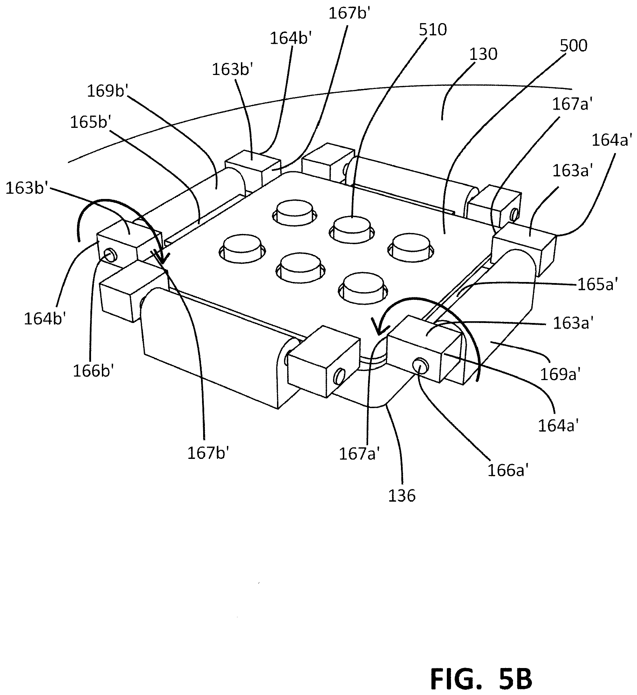

Another embodiment of rotary positioning elements is shown in **FIG.5A** and **FIG.5B****.** In contrast with the embodiment of **FIG.4A** and **FIG.4B** described immediately above, the horizontal positioning and vertical positioning of a nest between two mutually counter-rotatable elements **164a'** and **164b'** in **FIG.5A** and **FIG.5B** are mutually independent positioning actions. This is achieved by employing, in each of the two mutually perpendicular planar dimensions addressed in the embodiment immediately above, a pair of fixed opposing planar tabs **165a'** and **165b'** to position nest **500** in the vertical dimension, and a pair of rotary positioning elements **164a'** and **164b'** to position nest **500** in the first horizontal dimension. In this embodiment, each of elements **164a'** and **164b'** comprise two rotatable elements ganged on axles **166a'** and **166b'** respectively to rotate in unison and mutual alignment either side of planar tabs **165a'** and **165b'** within bosses **169a'** and **169b'** respectively. The sets of rotary elements **164a'** and **164b',** beyond each being divided in to two ganged elements, serve to confine nest **500** in the horizontal dimension in the same fashion as rotary elements **164a** and **164b** in the embodiment of **FIG.4A** and **FIG.4B** described immediately above.

While elements **164a'** and **164b'** may be designed to be of more complex shape, we show in **FIG.5A** and **FIG.5B** a very simple implementation in which surfaces **167a'** of rotary elements **164a'** and surfaces **167b'** of rotary elements **164b'** serve to position nest **500** in the first horizontal dimension. By rotating elements **164a'** joined by axle **166a'** counter-clockwise within boss **169a'** and rotating elements **164b'** joined by axle **166b'** clockwise within boss **169b',** Surfaces **163a'** and **163b'** may be made to face each other and thereby a nest of different length in the first horizontal dimension may be positioned and accurately located between elements **164a'** and **164b'.**

Ganged elements **164a'** and **164b'** may be rotated manually about the axes of axles **166a'** and **166b'** respectively inside bosses **169a'** and **169b'** respectively. In some embodiments, the rotation of elements **164a'** and **164b'** may be done automatically by means of motorized drives controlled by controller **400** and suitable control software. That control may be based on predetermined dimensional data relating to the nest being positioned between the surfaces of elements **164a'** and **164b'.** It may also be based on input data derived from imaging data obtained from cameras **210** and/or **220.** Further, the rotation may take place as nest **500** is lowered into position so that the particular surfaces of elements **164a'** and **164b'** destined to engage with the opposing ends of nest **500** along the first dimension may serve as closing horizontal grip on nest **500** as the surfaces rotate toward the position in which they face each other.

**FIG.5A** and **FIG.5B** show a further set of paired mutually counter-rotatable rotary positioning elements, not numbered for the sake of clarity, ganged similarly to rotary elements **164a'** and **164b',** and disposed to accurately locate nest **500** independently in the vertical dimension and in a second planar dimension of nest **500** perpendicular to the first dimension.

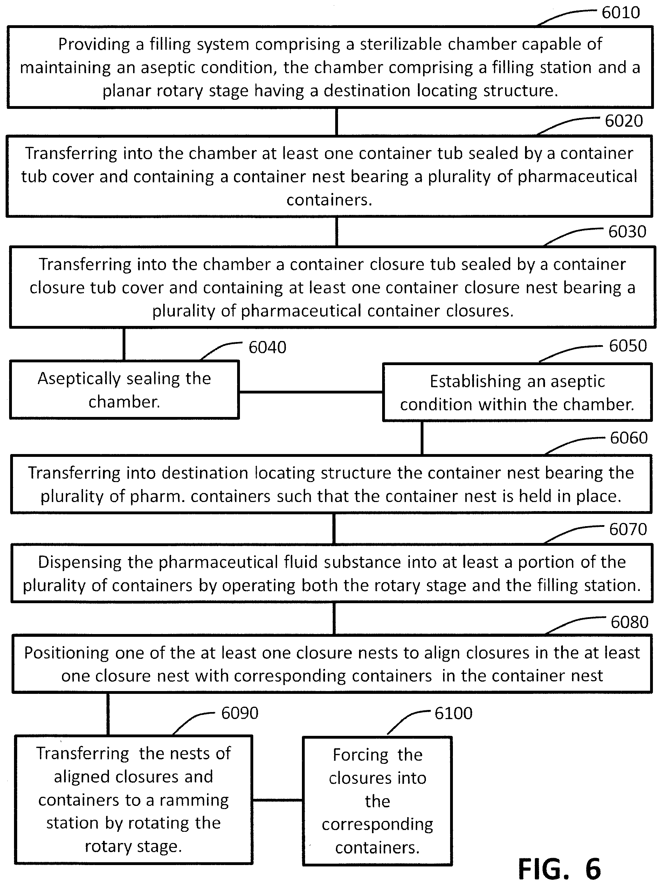

In a further aspect, described at the hand of **FIG.6****,** a method is provided for filling nested pharmaceutical containers **510** with a pharmaceutical fluid substance, the method comprising: providing **[6010]** filling system **1000** comprising sterilizable chamber **100** capable of maintaining an aseptic condition, chamber **100** comprising filling station **170** and planar rotary stage **130** having destination locating structure **136, 164a, 164b, 164a', 164b';** transferring **[6020]** into the chamber at least one container tub **530** sealed by container tub cover **520** and containing container nest **500** bearing a plurality of pharmaceutical containers **510;** aseptically sealing **[6040]** chamber **100;** establishing **[6050]** an aseptic condition within chamber **100;** transferring **[6060]** into destination locating structure **136, 164a, 164b, 164a', 164b'** container nest **500** bearing the plurality of pharmaceutical containers **510** such that container nest **500** is held in place; and dispensing **[6070]** the pharmaceutical fluid substance into at least a portion of the plurality of pharmaceutical containers **510** by operating both rotary stage **130** and filling station **170.** Operating filling station **170** may include rotating filling station **170.** Dispensing the pharmaceutical fluid substance may comprise dispensing the pharmaceutical fluid substance on an iterative and serial basis into containers **510.**

Providing **[6010]** filling system **1000** may comprise providing filing apparatus comprising at least one cover removal station **140** within chamber **100** and wherein the transferring into destination locating structure container tub **530** comprises removing container tub cover **520** from container tub **530** by operating both rotary stage **130** and the at least one cover removal station **140.** Operating the at least one cover removal station **140** may comprise rotating the at least one cover removal station **140.** Providing **[6010]** filling system **1000** may comprise providing within chamber **100** at least one cover removal station **140** having engagement tool **142,** transferring **[6020]** into chamber **100** at least one container tub **530** may comprise attaching to container tub **520** cover, cover removal fixture **540;** and wherein the operating the at least one cover removal station **140** comprises engaging engagement tool **142** with cover removal fixture **540.**

The method may further comprise transferring **[6030]** into the chamber container closure tub **630** sealed by a container closure tub cover and containing at least one container closure nest **600** bearing a plurality of pharmaceutical container closures **610.** The method may further comprise positioning **[6080]** one of the at least one closure nests **600** to align closures **610** in the at least one closure nest **600** with corresponding containers **530** in container nest **500;** transferring **[6090]** nests **500,600** of aligned closures **610** and containers **510** to a ramming station by rotating the rotary stage **130;** and forcing **[6100]** closures **610** into corresponding containers **510.** The method may further include adjusting tub locating structure **135,145** to accommodate a size of closure nest tub **630.** Positioning **[6080]** one of the at least one closure nest **600** may comprise: obtaining image information about the one of the at least one closure nests **600;** and positioning the one of the at least one closure nests **600** based on the image information. Positioning **[6080]** one of the at least one closure nest **600** may comprise: applying a vacuum to suction cups **162, 152a, 152b, 152a', 152b';** lifting container closure nest **600** with the suction cups; and operating rotary stage **130.**

Transferring **[6020]** into destination locating opening container nest **500** may comprise: applying a vacuum to the suction cups; lifting container nest **500** with the suction cups; and operating rotary stage **130.** The method may further include selecting one of a plurality of sets of suction cups and wherein the applying a vacuum to suction cups is performed for the selected set of suction cups. The selecting may include rotating one of the plurality of sets of suction cups into position. The method may further include destination locating structure **136, 164a, 164b, 164a', 164b'** to accommodate a size of container nest **500.** Adjusting may be performed in two at least generally orthogonal directions. The method may further include adjusting tub locating structure **139,149** to accommodate a size of container nest tub **530.**

In a further aspect, a method is provided (see **FIG.1G****)** for removing within a controlled environment enclosure a container cover from a sealed container, for example tub **530** or tub **630,** the sealed container being sealed by the container cover, for example cover **520,** the method comprising: providing the container in controlled environment enclosure **100** with cover **520** sealed to a sealing surface of a lip of the container to seal the contents of the container against decontamination, cover **520** having cover removal fixture **540,** decontaminating the sealed container in controlled environment enclosure **100,** engaging cover removal fixture **540** with engagement tool **142,** and removing the cover from the container using engagement tool **142.** Engaging may engage cover removal fixture **540** with fork-shaped engagement tool **142.** Engaging may engage a ball-shaped appendage on cover removal fixture **540.**

Providing may include providing sterilized pharmaceutical containers **510** or closures **610** in the sealed container, for example tub **530** or **630,** before the decontaminating. Attaching may take place before the container is in the controlled environment enclosure **100.** Decontaminating the sealed container in controlled environment enclosure **100** may take place before the removing of cover **520.** Removing cover **520** may include moving engagement tool **142** relative to container **530.** Removing cover **520** may include moving both container **530** and engagement tool **142.** The method may further comprise attaching cover removal fixture **540** to cover **520** before providing container **530** in the controlled environment enclosure.

## Claims

1. A system (1000) for filling nested pharmaceutical containers (510) with a pharmaceutical fluid substance, the system comprising a sterilizable chamber capable of maintaining an aseptic condition, the chamber comprising:
a planar rotary stage (130) having a rotary stage rotation axis (131),
a plurality of locating structures (132, 134, 135, 136, 139) positioned on the surface of the rotary stage at different positions around the rotary stage rotation axis, for holding nests (500, 600) of pharmaceutical container parts (510, 610) at the different positions around the rotary stage rotation axis (131), wherein at least one of the locating structures includes a reconfigurable locating structure with one or more adjustable positioning surfaces to position a tub (530, 630) with respect to the rotary stage (130), and
a container filling station (170) having a dispensing head for filling the containers (510) while they are held in a nest (500) at one of the locating structures.

2. The system of claim 1 wherein the locating structures include surfaces associated with a first tub-holding opening in the rotary stage for holding a first tub containing at least one nest of containers, surfaces associated with a second tub-holding opening in the rotary stage for holding a second tub containing at least one nest of closures, and surfaces associated with a destination nest-holding opening in the rotary stage for holding at least one nest.

3. The system of claim 1 or 2 wherein the chamber further comprises at least one vacuum pickup system comprising suction cups disposed to engage with the container nest and container closure nest held on the rotary stage, the at least one vacuum pickup system being configured in combination with rotation of the rotary stage to lift a pharmaceutical container nest from a pharmaceutical container tub and to deposit the pharmaceutical container nest in the destination opening in combination with rotation of the rotary stage and to lift a pharmaceutical container closure nest from a pharmaceutical container closure tub and to deposit the container closure nest on top of the pharmaceutical container nest.

4. The system of any of claims 1-3 wherein the reconfigurable locating structure includes at least one pair of a reconfigurable stopping member and a restraining member disposed opposite each other across an opening in the rotary stage to precisely position at a first predetermined position a tub that contains at least one nest.

5. The system of any of claims 1-4 wherein the stopping member is adjustable to stop the tub at the first predetermined position by a rotary adjustment and the restraining member is disposed to restrain the tub in the first predetermined position.

6. The system of any of claims 1-5 wherein at least a first of the reconfigurable locating structures includes a rotary positioning element having an axis of rotation parallel to a plane of the rotary stage and includes a plurality of different positioning surfaces that are selectable by rotating the rotary positioning element.

7. The system of any of claims 1-6 wherein at least one of the reconfigurable locating structures includes a pair of opposing rotary positioning elements each having an axis of rotation parallel to a plane of the rotary stage and each including a plurality of different positioning surfaces that are selectable by rotating that rotary positioning elements, to accommodate different nest widths.

8. The system of any of claims 1-7 wherein at least one of the reconfigurable locating structures includes at least a first pair of opposing positioning elements that define positioning surfaces that oppose each other along a first positioning axis that is at least generally parallel to a plane of the rotary stage and at least a second pair of opposing positioning elements that define positioning surfaces that oppose each other along a second positioning axis that is at least generally parallel to a plane of the rotary stage and at least generally perpendicular to the first positioning axis.

9. The system of any of claims 1-8 further including a reconfigurable vacuum pickup system comprising:
a first set of suction cups arranged in a first pattern,
a second set of suction cups arranged in a second pattern different from the first pattern, and a selection mechanism operative to position either the first set of suction cups or the second set of suction cups to engage with the at least a first of the nests of pharmaceutical container parts while it is held by one of the plurality of locating structures.

10. The system of any of claims 1-9 wherein the selection mechanism of the reconfigurable vacuum pickup system includes a rotary mechanism operative to position the first or second sets of suction cups in an engagement position.

11. The system of any of claims 1-10 further including at least one cover removal station positioned to remove tub covers from tubs containing at least one nest of pharmaceutical packaging materials held in one of the locating structures.

12. The system of any of claims 1-11 further comprising at least one camera disposed to obtain image information about at least one of the nests of pharmaceutical container parts.

13. The system of any of claims 1-12 further comprising:
at least one camera for obtaining image information of at least one of the container nest and the closure nest,
a controller comprising a memory and a processor, and
wherein the controller is operative to instruct the rotary stage to rotate to angular positions that are one of predetermined and based on the image information and to control the at least one cover removal station, the filling station, the at least one vacuum pickup system, and the ram system to operate in conjunction with the rotary stage.

14. In a device (1000) specially adapted for bringing pharmaceutical products into particular physical or administering forms, a method for filling nested pharmaceutical containers (510) with a pharmaceutical fluid substance, the method comprising:
providing a filling system comprising a sterilizable chamber capable of maintaining an aseptic condition, the chamber comprising a filling station (170) and a planar rotary stage (130) having a rotary stage rotation axis (131) and a destination fiducial locating structure,
wherein a plurality of locating structures (132, 134, 135, 136, 139) are positioned on the surface of the rotary stage at different positions around the rotary stage rotation axis, for holding nests (600) of pharmaceutical container parts (510, 610) at the different positions around the rotary stage rotation axis, and
wherein at least one of the locating structures includes a reconfigurable locating structure with one or more adjustable positioning surfaces to position a tub (530, 630) with respect to the rotary stage;
transferring into the chamber a plurality of container tubs (530) each sealed by a container tub cover (630) and each containing a container nest (500) bearing a plurality of pharmaceutical containers;
aseptically sealing the chamber;
establishing an aseptic condition within the chamber;
transferring into the destination fiducial locating structure the container nest (500) bearing the plurality of pharmaceutical containers (510) such that the container nest (500) is held in place by the positioning surfaces, the pharmaceutical containers including a bottom, a top lip, and sidewalls having a peelable container cover with a cover removal fixture sealed to the top lip;
dispensing the pharmaceutical fluid substance into at least a portion of the plurality of pharmaceutical containers (510) by operating both the rotary stage (130) and the filling station (170) in relation to the locating structure; and
monitoring the closure status of at least one container tub cover (630) after the dispensing step

## Patentansprüche

1. System (1000) zum Füllen von in einem Nest angeordneten pharmazeutischen Behältern (510) mit einer pharmazeutischen flüssigen Substanz, wobei das System eine sterilisierbare Kammer umfasst, die in der Lage ist, einen aseptischen Zustand aufrechtzuerhalten, wobei die Kammer Folgendes umfasst:
einen ebenen Drehtisch (130) mit einer Drehtisch-Drehachse (131),
eine Vielzahl von Fixierstrukturen (132, 134, 135, 136, 139), die auf der Oberfläche des Drehtisches an verschiedenen Positionen um die Drehtisch-Drehachse herum angeordnet sind, um Nester (500, 600) von pharmazeutischen Behälterteilen (510, 610) an den verschiedenen Positionen um die Drehtisch-Drehachse (131) herum zu halten, wobei mindestens eine der Fixierstrukturen eine rekonfigurierbare Fixierstruktur mit einer oder mehreren einstellbaren Positionierungsflächen zum Positionieren einer Wanne (530, 630) in Bezug auf den Drehtisch (130) und eine Behälterfüllstation (170) mit einem Ausgabekopf zum Füllen der Behälter (510) einschließt, während sie in einem Nest (500) an einer der Fixierstrukturen gehalten werden.

2. System nach Anspruch 1, wobei die Fixierstrukturen Oberflächen, die mit einer ersten Wannenhalteöffnung in dem Drehtisch verbunden sind, um eine erste Wanne zu halten, die mindestens ein Nest von Behältern enthält, Oberflächen, die mit einer zweiten Wannenhalteöffnung in dem Drehtisch verbunden sind, um eine zweite Wanne zu halten, die mindestens ein Nest von Verschlüssen enthält, und Oberflächen einschließen, die mit einer Bestimmungsnest-Halteöffnung in dem Drehtisch verbunden sind, um mindestens ein Nest zu halten.

3. System nach Anspruch 1 oder 2, wobei die Kammer weiter mindestens ein Vakuumaufnahmesystem umfasst, das Saugnäpfe umfasst, die so angeordnet sind, dass sie mit dem auf dem Drehtisch gehaltenen Behälternest und Behälterverschlussnest in Eingriff kommen, wobei das mindestens eine Vakuumaufnahmesystem in Kombination mit der Drehung des Drehtisches konfiguriert ist, um ein pharmazeutisches Behälternest aus einer pharmazeutischen Behälterwanne zu heben und das pharmazeutische Behälternest in der Bestimmungsöffnung in Kombination mit der Drehung des Drehtisches abzulegen und ein pharmazeutisches Behälterverschlussnest aus einer pharmazeutischen Behälterverschlusswanne zu heben und das Behälterverschlussnest auf dem pharmazeutischen Behälternest abzulegen.

4. System nach einem der Ansprüche 1-3, wobei die rekonfigurierbare Fixierstruktur mindestens ein Paar aus einem rekonfigurierbaren Stoppelement und einem Rückhalteelement einschließt, die einander gegenüberliegend über einer Öffnung in dem Drehtisch angeordnet sind, um eine Wanne, die mindestens ein Nest enthält, präzise an einer ersten vorbestimmten Position zu positionieren.

5. System nach einem der Ansprüche 1-4, wobei das Stoppelement einstellbar ist, um die Wanne in der ersten vorbestimmten Position durch eine Dreheinstellung anzuhalten, und das Rückhalteelement so angeordnet ist, dass es die Wanne in der ersten vorbestimmten Position zurückhält.

6. System nach einem der Ansprüche 1-5, wobei mindestens eine erste der rekonfigurierbaren Fixierstrukturen ein drehbares Positionierungselement mit einer Drehachse parallel zu einer Ebene des Drehtisches einschließt und eine Vielzahl unterschiedlicher Positionierungsflächen einschließt, die durch Drehen des drehbaren Positionierungselements ausgewählt werden können.

7. System nach einem der Ansprüche 1-6, wobei mindestens eine der rekonfigurierbaren Fixierstrukturen ein Paar gegenüberliegender drehbarer Positionierungselemente einschließt, die jeweils eine Drehachse parallel zu einer Ebene des Drehtisches aufweisen und jeweils eine Vielzahl unterschiedlicher Positionierungsflächen einschließen, die durch Drehen der drehbaren Positionierungselemente ausgewählt werden können, um unterschiedliche Nestbreiten aufzunehmen.

8. System nach einem der Ansprüche 1-7, wobei mindestens eine der rekonfigurierbaren Fixierstrukturen mindestens ein erstes Paar gegenüberliegender Positionierungselemente einschließt, die Positionierungsflächen definieren, die einander entlang einer ersten Positionierungsachse gegenüberliegen, die zumindest im Allgemeinen parallel zu einer Ebene des Drehtisches ist, und mindestens ein zweites Paar gegenüberliegender Positionierungselemente, die Positionierungsflächen definieren, die einander entlang einer zweiten Positionierungsachse gegenüberliegen, die zumindest im Allgemeinen parallel zu einer Ebene des Drehtisches und zumindest im Allgemeinen senkrecht zu der ersten Positionierungsachse ist.

9. System nach einem der Ansprüche 1-8, weiter einschließend ein rekonfigurierbares Vakuumaufnahmesystem, das Folgendes umfasst:
einen ersten Satz von Saugnäpfen, die nach einem ersten Muster angeordnet sind,
einen zweiten Satz von Saugnäpfen, die in einem zweiten Muster angeordnet sind, das sich von dem ersten Muster unterscheidet, und
einen Auswahlmechanismus, der wirksam ist, um entweder den ersten Satz von Saugnäpfen oder den zweiten Satz von Saugnäpfen so zu positionieren, dass sie mit mindestens einem ersten der Nester von pharmazeutischen Behälterteilen in Eingriff kommen, während es von einer der Vielzahl von Fixierstrukturen gehalten wird.

10. System nach einem der Ansprüche 1-9, wobei der Auswahlmechanismus des rekonfigurierbaren Vakuumaufnahmesystems einen Drehmechanismus einschließt, der dazu dient, den ersten oder zweiten Satz von Saugnäpfen in einer Eingriffsposition zu positionieren.

11. System nach einem der Ansprüche 1-10, weiter einschließend mindestens eine Abdeckungsentfernungsstation, die zum Entfernen von Wannenabdeckungen von Wannen positioniert ist, die mindestens ein Nest aus pharmazeutischem Verpackungsmaterial enthalten, das in einer der Fixierstrukturen gehalten wird.

12. System nach einem der Ansprüche 1-11, weiter umfassend mindestens eine Kamera, die so angeordnet ist, dass sie Bildinformationen über mindestens eines der Nester mit pharmazeutischen Behälterteilen erhält.

13. System nach einem der Ansprüche 1-12, weiter umfassend:
mindestens eine Kamera zum Erhalten von Bildinformationen von mindestens einem von dem Behälternest und dem Verschlussnest,
eine Steuereinheit, die einen Speicher und einen Prozessor umfasst, und
wobei die Steuereinheit so arbeitet, dass sie den Drehtisch anweist, sich in Winkelpositionen zu drehen, die entweder vorbestimmt sind oder auf der Bildinformation basieren, und die mindestens eine Abdeckungsentfernungsstation, die Füllstation, das mindestens eine Vakuumaufnahmesystem und das Stempelsystem so steuert, dass sie in Verbindung mit dem Drehtisch arbeiten.

14. Verfahren zum Befüllen von in einem Nest angeordneten pharmazeutischen Behältern (510) mit einer pharmazeutischen flüssigen Substanz in einer Vorrichtung (1000), die speziell dafür ausgelegt ist, pharmazeutische Produkte in bestimmte physische oder Verabreichungsformen zu bringen, wobei das Verfahren Folgendes umfasst:
Bereitstellen eines Abfüllsystems, das eine sterilisierbare Kammer umfasst, die in der Lage ist, einen aseptischen Zustand aufrechtzuerhalten, wobei die Kammer eine Füllstation (170) und einen ebenen Drehtisch (130) mit einer Drehtisch-Drehachse (131) und einer Bestimmungsreferenz-Fixierstruktur umfasst,
wobei eine Vielzahl von Fixierstrukturen (132, 134, 135, 136, 139) auf der Oberfläche des Drehtisches an verschiedenen Positionen um die Drehtisch-Drehachse positioniert sind, um Nester (600) von pharmazeutischen Behälterteilen (510, 610) an den verschiedenen Positionen um die Drehtisch-Drehachse zu halten, und
wobei mindestens eine der Fixierstrukturen eine rekonfigurierbare Fixierstruktur mit einer oder mehreren einstellbaren Positionierungsflächen einschließt, um eine Wanne (530, 630) in Bezug auf den Drehtisch zu positionieren;
Überführen einer Vielzahl von Behälterwannen (530) in die Kammer, wobei jede durch eine Behälterwannenabdeckung (630) versiegelt ist und jede ein Behälternest (500) enthält, das eine Vielzahl von pharmazeutischen Behältern trägt;
aseptisches Abdichten der Kammer;
Herstellen eines aseptischen Zustands innerhalb der Kammer;
Überführen des Behälternestes (500), das die Vielzahl von pharmazeutischen Behältern (510) trägt, in die Bestimmungsreferenz-Fixierstruktur, so dass das Behälternest (500) durch die Positionierungsflächen an Ort und Stelle gehalten wird, wobei die pharmazeutischen Behälter einen Boden, eine Oberlippe und Seitenwände mit einer abziehbaren Behälterabdeckung mit einer Abdeckungsentfernungseinrichtung, die mit der Oberlippe versiegelt ist, einschließen;
Abgeben der pharmazeutischen flüssigen Substanz in mindestens einen Teil der Vielzahl von pharmazeutischen Behältern (510) durch Betreiben sowohl des Drehtisches (130) als auch der Füllstation (170) in Bezug auf die Fixierstruktur; und
Überwachen des Verschlusszustands mindestens einer Behälterwannenabdeckung (630) nach dem Abgabeschritt.

## Revendications

1. Système (1000) de remplissage de récipients pharmaceutiques (510) emboîtés renfermant une substance fluide pharmaceutique, le système comprenant une chambre stérilisable en mesure de maintenir une condition aseptique, la chambre comprenant :
une platine rotative (130) planaire présentant un axe de rotation de platine rotative (131),
une pluralité de structures de localisation (132, 134, 135, 136, 139) positionnées à la surface de la platine rotative à différentes positions autour de l'axe de rotation de platine rotative, pour retenir des emboîtements (500, 600) de parties de récipient pharmaceutique (510, 610) aux différentes positions autour de l'axe de rotation de platine rotative (131), dans lequel au moins une des structures de localisation inclut une structure de localisation reconfigurable avec une ou plusieurs surfaces de positionnement réglables pour positionner un bac (530, 630) par rapport à la platine rotative (130), et
une station de remplissage de récipient (170) présentant une tête de distribution pour le remplissage des récipients (510) lorsqu'ils sont retenus dans un emboîtement (500) au niveau de l'une des structures de localisation.

2. Système selon la revendication 1, dans lequel les structures de localisation incluent des surfaces associées à une ouverture de retenue de premier bac dans la platine rotative pour retenir un premier bac contenant au moins un emboîtement de récipients, des surfaces associées à une ouverture de retenue de second bac dans la platine rotative pour retenir un second bac contenant au moins un emboîtement de fermetures, et des surfaces associées à une ouverture de retenue d'emboîtement de destination dans la platine rotative pour retenir au moins un emboîtement.

3. Système selon la revendication 1 ou la revendication 2, dans lequel la chambre comprend en outre au moins un système de prélèvement sous vide comprenant des ventouses disposées de manière à venir en prise avec l'emboîtement de récipient et l'emboîtement de fermeture de récipient retenus sur la platine rotative, le au moins un système de prélèvement sous vide étant configuré en combinaison avec la rotation de la platine rotative pour lever un emboîtement de récipient pharmaceutique à partir d'un bac de récipient pharmaceutique et pour déposer l'emboîtement de récipient pharmaceutique dans l'ouverture de destination en combinaison avec la rotation de la platine rotative et pour lever un emboîtement de fermeture de récipient pharmaceutique à partir d'un bac de fermeture de récipient pharmaceutique et pour déposer l'emboîtement de fermeture de récipient sur le dessus de l'emboîtement de récipient pharmaceutique.

4. Système selon l'une quelconque des revendications 1 à 3, dans lequel la structure de localisation reconfigurable inclut au moins une paire d'un élément d'arrêt reconfigurable et d'un élément de retenue disposés de manière opposée l'un par rapport à l'autre à travers une ouverture dans la platine rotative pour positionner précisément au niveau d'une première position prédéterminée un bac qui contient au moins un emboîtement.

5. Système selon l'une quelconque des revendications 1 à 4, dans lequel l'élément de retenue est réglable pour arrêter le bac au niveau de la première position prédéterminée par un réglage rotatif et l'élément de retenue est disposé pour retenir le bac dans la première position prédéterminée.

6. Système selon l'une quelconque des revendications 1 à 5, dans lequel au moins une première des structures de localisation reconfigurables inclut un élément de positionnement rotatif présentant un axe de rotation parallèle à un plan de la platine rotative et inclut une pluralité de différentes surfaces de positionnement qui peuvent être sélectionnées par rotation de l'élément de positionnement rotatif.

7. Système selon l'une quelconque des revendications 1 à 6, dans lequel au moins une des structures de localisation reconfigurables inclut une paire d'éléments de positionnement rotatifs opposés présentant chacun un axe de rotation parallèle à un plan de la platine rotative et incluant chacun une pluralité de différentes surfaces de positionnement qui peuvent être sélectionnées par rotation de ces éléments de positionnement rotatifs, afin de loger différentes largeurs d'emboîtement.

8. Système selon l'une quelconque des revendications 1 à 7, dans lequel au moins une des structures de localisation reconfigurables inclut au moins une première paire d'éléments de positionnement opposés qui définissent des surfaces de positionnement qui s'opposent l'une à l'autre le long d'un premier axe de positionnement qui est au moins généralement parallèle à un plan de la platine rotative et au moins une seconde paire d'éléments de positionnement opposés qui définissent des surfaces de positionnement qui s'opposent l'une à l'autre le long d'un second axe de positionnement qui est au moins généralement parallèle à un plan de la platine rotative et au moins généralement perpendiculaire au premier axe de positionnement.

9. Système selon l'une quelconque des revendications 1 à 8, incluant en outre un système de prélèvement sous vide reconfigurable comprenant :
un premier ensemble de ventouses agencées selon un premier motif,
un second ensemble de ventouses agencées selon un second motif différent du premier motif, et
un mécanisme de sélection fonctionnel pour positionner soit le premier ensemble de ventouses soit le second ensemble de ventouses pour venir en prise avec le au moins un premier des emboîtements de parties de récipient pharmaceutique lorsqu'il est retenu par une de la pluralité de structures de localisation.

10. Système selon l'une quelconque des revendications 1 à 9, dans lequel le mécanisme de sélection du système de prélèvement sous vide reconfigurable inclut un mécanisme rotatif fonctionnel pour positionner le premier ou le second ensemble de ventouses dans une position de mise en prise.

11. Système selon l'une quelconque des revendications 1 à 10, incluant en outre au moins une station de retrait de couvercle positionnée de manière à retirer des couvercles de bac de bacs contenant au moins un emboîtement de matériaux d'emballage pharmaceutique retenu dans l'une des structures de localisation.

12. Système selon l'une quelconque des revendications 1 à 11, comprenant en outre au moins une caméra disposée pour obtenir des informations d'image sur au moins l'un des emboîtements de parties de récipient pharmaceutique.

13. Système selon l'une quelconque des revendications 1 à 12, comprenant en outre :
au moins une caméra pour obtenir des informations d'image d'au moins un de l'emboîtement de récipient et de l'emboîtement de fermeture,
un dispositif de commande comprenant une mémoire et un processeur, et
dans lequel le dispositif de commande est fonctionnel pour ordonner à la platine rotative de tourner vers des positions angulaires qui sont l'une de positions angulaires prédéterminées et de positions angulaires sur la base des informations d'image et pour commander la au moins une station de retrait de couvercle, la station de remplissage, le au moins un système de prélèvement sous vide, et le système de vérin de manière à fonctionner conjointement avec la platine rotative.

14. Dans un dispositif (1000) spécialement adapté pour mettre des produits pharmaceutiques sous des formes physiques ou d'administration particulières, procédé de remplissage de récipients pharmaceutiques emboîtés (510) renfermant une substance fluide pharmaceutique, le procédé comprenant :
la fourniture d'un système de remplissage comprenant une chambre stérilisable en mesure de maintenir une condition aseptique, la chambre comprenant une station de remplissage (170) et une platine rotative (130) planaire présentant un axe de rotation de platine rotative (131) et une structure de localisation de repère de destination,
dans lequel une pluralité de structures de localisation (132, 134, 135, 136, 139) sont positionnées à la surface de la platine rotative à différentes positions autour de l'axe de rotation de platine rotative, pour retenir des emboîtements (600) de parties de récipient pharmaceutique (510, 610) aux différentes positions autour de l'axe de rotation de platine rotative, et
dans lequel au moins une des structures de localisation inclut une structure de localisation reconfigurable avec une ou plusieurs surfaces de positionnement réglables pour positionner un bac (530, 630) par rapport à la platine rotative ;
le transfert dans la chambre d'une pluralité de bacs de récipient (530) chacun étanché par un couvercle de bac de récipient (630) et chacun contenant un emboîtement de récipient (500) portant une pluralité de récipients pharmaceutiques ;
l'étanchement aseptique de la chambre ;
l'établissement d'une condition aseptique à l'intérieur de la chambre ;
le transfert dans la structure de localisation de repère de destination de l'emboîtement de récipient (500) portant la pluralité de récipients pharmaceutiques (510) de sorte que l'emboîtement de récipient (500) soit retenu en place par les surfaces de positionnement, les récipients pharmaceutiques incluant un fond, une lèvre supérieure, et des parois présentant un couvercle de récipient pelable avec une fixation de retrait de couvercle étanchée à la lèvre supérieure ;
la distribution de la substance fluide pharmaceutique dans au moins une portion de la pluralité de récipients pharmaceutiques (510) en faisant fonctionner à la fois la platine rotative (130) et la station de remplissage (170) par rapport à la structure de localisation ; et
la surveillance de l'état de fermeture d'au moins un couvercle de bac de récipient (630) après l'étape de distribution.
